# EUROPEAN PATENT APPLICATION

(11) **EP 2 390 253 A1**
(43) Date of publication of application: **30.11.2011**
(21) Application number: 10164258.5
(22) Date of filing: 28.05.2010
(51) Int. Cl.: C07D 471/06, C09B 5/00, H01L 51/00

(54) **Morylenes, a new class of modified rylenes**

(71) Applicant: Universität des Saarlandes, 66123 Saarbrücken (DE)
(72) Inventor: Sachdev, Hermann, 66123, Saarbrücken (DE)
(74) Representative: von Kreisler Selting Werner

(57) **Abstract**

The present invention provides hitherto unknown pyrene- and rylene-type compounds of formulas (I) to (IV), a method for their production and their use, notably as fluorescent dye and as intermediate for modifying pyrenes and rylenes or related chromophores.

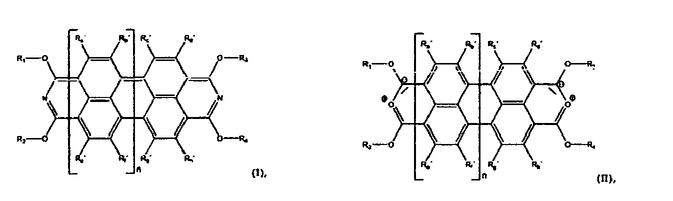

## Description

The present invention provides hitherto unknown pyrene- and rylene-type compounds, a method for their production and their use, notably as fluorescent dye and as intermediate for modifying pyrenes and rylenes or related chromophores.

### Background of the Invention

Many fluorescent dyes are based upon perylene and related systems, containing chromophores with a highly delocalised π-system. Functional rylene type dyes are of great technical importance and reveal a manifold of applications. Especially perylene derivatives reveal such a delocalised π-system and can be used as chromophores (H. Zollinger, Color Chemistry - Synthesis, Properties and Applications of Organic Dyes and Pigments, 3rd ed., Helv. Chim. Acta, Zürich (2003); F. Würthner, Chem. Com., 1564-1579 (2004); Y. Avlasevich, K. Müllen, Chem. Com., 4440-4442 (2006); K. Peneva et al., Angew. Chemie Int. Ed. 47, 3289 - 3467 (2008)). Usually rylene-type dyes can be functionalized either at the imide position by a reaction of various amines and the corresponding carbonic acid anhydride or by substitution of the hydrogen atoms with other functional groups. Various methods of derivatizations are known in the art and concern specific modifications of the rylene compounds towards emission and absorption behaviour, their chemical reactivity and their solubility, which can currently only by controlled by selective functionalizations at the imide nitrogen atoms as well as the aromatic core structure (e.g. H. Langhals, Helvetica Chimica Acta 88, 1309-1343 (2005)). Scheme 1 shows the typical structure of a rylene type fluorescent dye. A general disadvantage of these systems is their limited solubility, which is currently enhanced by alterations of the amine (marked as position A), e.g. by introducing sterically demanding substituents, or by modifications of the core structure (marked as position B). So far, there are no reports about a modification of the carbonyl function combined with a change of the electronic structure of the imido- units (marked as C). 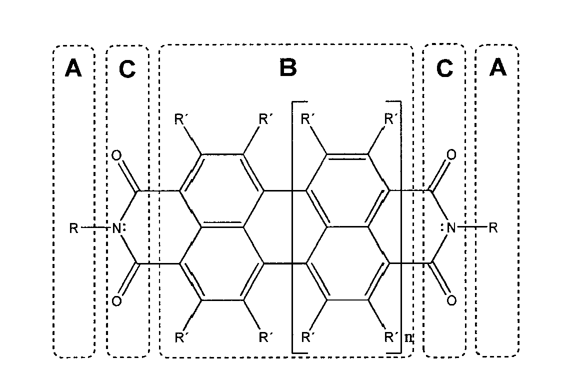 Scheme 1: Typical structure and current methods of functionalization of rylene type fluorescent dyes. The systems are based on the following core structures (n=0 naphthalinetetracarbonic acid diimide NTCDI, n = 1 perylenetetracarbonic acid diimide PCTDI; n = 2 terrylenetetracarbonic acid diimide, n = 3 quaterrylenetetracarbonic acid diimide, n = 4 pentarylenetetracarbonic acid diimide, n = 5 hexarylenetetracarbonic acid diimide).

To obtain new dyes from these rylene type chromophores, they have to be functionalised. By linking electron acceptors or donors, the fluorescence and absorbtion properties of the dyes can be modified and improved (H. Langhals, Helvetica Chimica Acta, 88, 1309 - 1343 (2005); M. G. Debije et al., J. Mater. Chem. 15, 1270-1276 (2005)). This concept was already described by Kardos in 1913 (S. Demmig, H. Langhals, Chem. Ber., 121, 225-230 (1988)), and these dyes are known for their various applications and for their chemical stability.

Due to the perylene based chromophore and the functional imide groups, these molecules possess outstanding photochemical properties. For example perylenetetracarboxydiimides with different substituents at the nitrogen atom of the imide group reveal a very high photostability and form non-bleaching fluorescent pigments. The photostability and fluorescence quantum yield were determined for various substituents and quantum yields close to 100% could be achieved (Y. Avlasevich et al., J. Mater. Chem. (2010), DOI 10.1039/c000137f). Functional groups like nitrogen and oxygen atoms have proven to be good substituents, because they act as an electron donor or -acceptors and also provide bonding sites for substrates.

Naphthaline (NTCDI) - and perylenetetracarboxybisimides (PTCDI) are chemically easily accessible. These bisimides (see Scheme 1: R' = H, n = 0, 1) can be achieved from the corresponding tetracarboxybisanhydrides by condensation with ammonia or primary amines (H. Langhals et al., Eur. J. Org. Chem., 4313 - 4321 (2005); S.V. Bhosale et al., Chem. Soc. Rev. 37, 331 - 342 (2008)). Since the perylene chromophores provide a large π-electron system and polar carbonyl as well as NH- groups, intermolecular interactions like π-stacking or hydrogen bonds can occur, which may lead to agglomerations and superstructures. Because of such kind of interactions, these compounds can also be used as building blocks in supramolecular chemistry (A.J. Pollard et al., Angew. Chem. 122, 1838 -1843 (2010)).

In analogy to the naphtalenediimides, perylenediimides and related compounds are used in various fields for their photochemical properties. Perylendiimides are suitable organic n-semiconductors due to their high electron density and their high electron mobility and they may also serve as materials for field effect transistors (F. Würthner et al., J. Am. Chem. Soc. 126, 10611-10618 (2004)). The naphtalene analoges to perylene for example form different colored gels with the different isomeres, and they can also be used as sensors in the detection of DNS and pyrophosphate. Furthermore, OLEDs are also based on the optical properties of Perylenediimides (P. Ranke et al., Appl. Phys. Lett. 71, 1332-1334 (1997)). In addition, the π-electrons of the organic aromatic framework enable a π-stacking leading to self organized superstructures with π-π-stacking: These structures can function as nanoscale molecular cables in photo electronic devices and in nano electronics (F. Würthner et al., Chem. Eur. J. 2000, 6, 3871-3885 (200)). Perylene derivates can also be used as laser dyes (N. Sadrai, G.R. Bird, Optical Com., 51, 62-64 (1984)), and another application of the photochemical properties of the perylene dyes is their use as light harvesting systems coupled with solar cells (S. Erten et al., Eur. Phys. J. Appl. Phys. 36, 225-229 (2007)).

A striking feature of perylenetetracarboxydiimide - based dyes is their high fluorescence and high chemical stability due to the perylene chromophores. A major drawback of the rylene imides is their poor solubility in any common solvent. In order to enhance their solubility, an approach is to limit the influence of the polar imide groups (H. Langhals et al., Tetrahedron 56, 5435-5441(2000); S. Erten et al., Dyes and Pigment, 64, 171 - 178 (2005)) by bulky substituents at the nitrogen atoms and the core structure.

To find an optimal fluorescence dye based on perylene, a compromise between fluorescence quantum yield, chemical stability and solubility, as well as the simplest way of the synthesis must be found. In order to make this kind of dyes more soluble, the nitrogen atoms of the imide groups were substituted which lead to different results. Different substituents were synthesised and analysed (H. Langhals, Helvetica Chimica Acta 88, 1309 - 1343 (2005); M. G. Debije et al., J. Mater. Chem. 15, 1270-1276 (2005); Y. Avlasevich et al., J. Mater. Chem. (2010), DOI 10.1039/c000137f; S. Demmig, H. Langhals, Chem. Ber. 121, 225-230 (1988); H. Langhals et al., Eur. J. Org. Chem., 4313 - 4321 (2005)). Bulky aryl substituents had negative effects on the solubility, whereas long aliphatic chains led to an increased solubility in organic solvents. Due to the increased molecular weight the long aliphatic chains have a stronger effect on the specific extinction coefficient, which decreases as the molecular mass increases. Another way to make these systems more soluble is a derivatization of the halogenated perylene core structure and linking ligands with polar functional groups (Y. Avlasevich et al., J. Mater. Chem. (2010), DOI 10.1039/c000137f). The above mentioned properties of the perylenediimide dye make these compounds ideal for the described applications. Nevertheless, these compounds still show a poor solubility which limits their applications. A key problem in the chemistry of rylenoid systems is the limited range of accessible modifications of the core structures. Basically, all of the chromophores are functionalised either at the amine ligand by using long aliphatic ligands or special chromophores (see Scheme 1, A) or by modifications at the aromatic core structure (Scheme 1, B). So far there are no reports of a modification of the carbonyl functionality by enlarging the aromatic system.

### Summary of the Invention

It was now found that the carbonyl groups of the rylenes can be modified by a selective method which allows new types of functionalizations of these compounds. A reaction sequence was established that allows a straightforward modification of rylene type imides. A preparative route is described dealing with a basically inorganic way of increasing the solubility of rylene based chromophores. Reactive centers in rylene imides are represented by the carboxyl oxygen atoms. For example, a silylation carried out at these centres can shield the polar groups and silyl substituents introduced at these centers can lead to an increase in solubility, but also a change in the aromatic system occurs. Such silyl modified morylenes and their derivatives can serve as starting materials for the preparation of a manifold of new compounds, e.g. by treatment with electrophiles like borylating agents leading to borylated systems.

A new route allowing a straightforward synthesis of silylated morylenes was found and the properties of selected compounds are presented. The invention thus provides
(1) a pyrene- and rylene-type compound having the general formulas (I) to (IV) 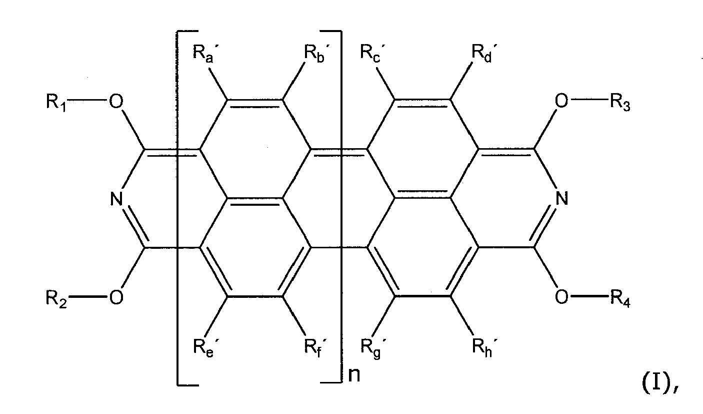 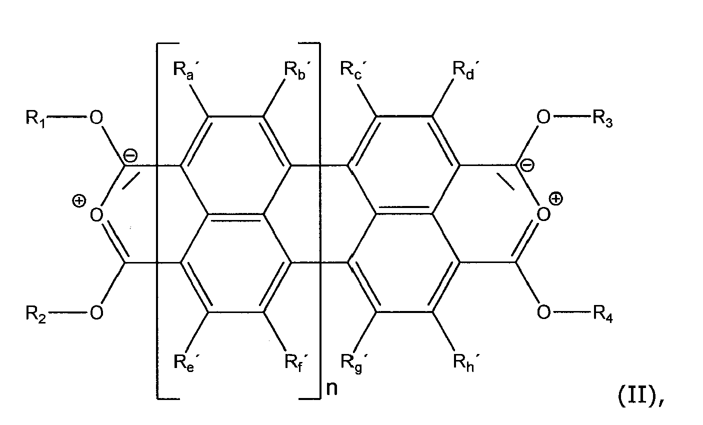 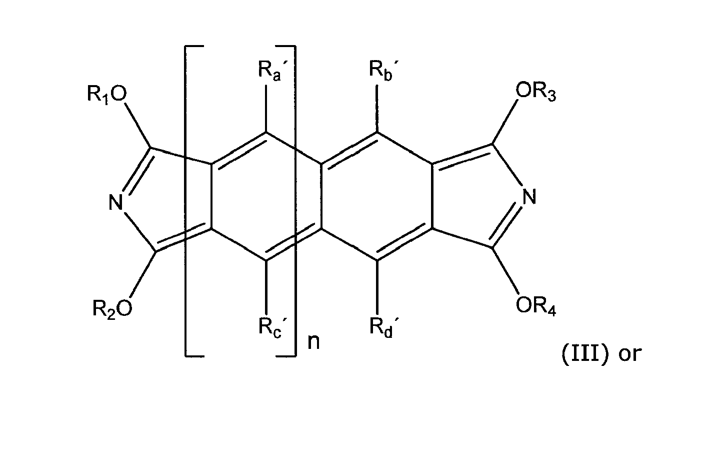 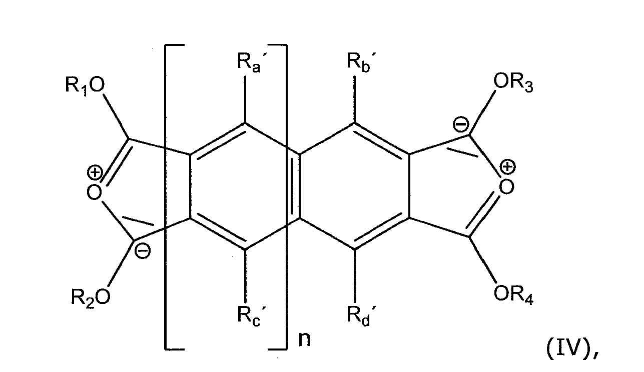 wherein
   n is an integer selected from 0 to 10,
   R₁ - R₄ is independently selected from an organosilyl group, an organoboryl group, an alkyl group, an aryl group, a metal ion, an organotin group, an organoalane group, an organozinc group, an organogermane group, an organogallane group, an organophosphorous group and a transition metal complex,
   R_{a'} - R_{h'} is at each occurrence independently selected from H, halogen, nitro, hydroxyl, an alkyl group, an aryl group, an alkyloxy group an aryloxy group, an alkylcarbonyl group, an arylcarbonyl group, a mercaptoalkyl group, a mercaptoaryl group, a carboxy group, a carbamide group, an alkyloxycarbonyl group, a thioester group, a vinylidene group, and an arylidene group, and
   Z and W shaped isomers thereof;
(2) a preferred embodiment of (1) above, where the compound has the formula (I) as shown in (1) above, n is 0 or 1, R_{a'} - R_{h'} are H, and R₁ - R₄ are trimethylsilyl groups;
(3) a method for preparing the pyrene- and rylene-type compound of formulas (I) to (IV) as defined in (1) or (2) above, which comprises
   (a) reacting starting compounds of the formulas (I) to (IV), wherein R is H, or a tautomer thereof, with a strong, alkali metal-containing base under reducing conditions, and,
   (b) if R₁ - R₄ are is other than a metal ion, reacting the product of step (a) with a minimum of appropriate equivalents of a silylating, borylating, alkylating, arylating, organotinylating, organoalanylating, organozincylatating, organogermanylating, organogallanylating, organophosphorylating and transition metal complex-forming agent;
(4) the use of a pyrene- and rylene-type compound of formulas (I) to (IV) as defined in (1) or (2) above as a fluorescent dye, as detectable marker in bioassays, as constituent of supramolecular systems and of self organisation systems, as reagent for electron transfer reactions and light harvesting systems, and as diagnostic tool for any species leading to a chemical degradation of the dye for a quantitative determination of such an agent (e.g. like water or oxygen or any other protic or oxidizing species); and
(5) the use of a pyrene- and rylene-type compound of formulas (I) to (IV) as defined in (1) or (2) above as intermediates for modification at positions that are vicinal to the carbon-oxygen groups of the pyrene- and rylene-type compounds of formulas (I) to (IV) or for modifying the N-atom of the compounds (I) to (II).

### Description of the Figures

Fig. 1: Packing diagram and molecular structure of compound (1) of the invention
Fig. 2: Packing diagram (view along c-axis) and molecular structure of compound (2) of the invention
Fig. 3: proposed NMR assignment of compounds (1) and (2) of the invention
Fig. 4: UV/Vis spectrum of compound (1) in dichloromethane
Fig. 5: UV/Vis spectrum of compound (2) in dichloromethane

### Detailed Description of the Invention

Aspect (1) of the invention pertains to pyrene- and rylene-type compounds of formulas (I) to (IV) as defined above (hereinafter also shortly referred to as compounds of the invention). As set forth above the residues R₁ - R₄ are independently selected from organosilyl groups, organoboryl groups, alkyl groups, aryl groups, organotin groups, organoalane groups, organozinc groups, organogermane groups, organogallane groups, organophosphorous groups, metal ions, and transition metal complexes, which means that the multiple residues R₁ - R₄ in the compounds of the invention can be same ore different.

Alkyl groups (hereinafter also referred to as "Alk"-groups) within the meaning of the invention include unsubstituted and substituted, saturated and unsaturated, linear, branched and cyclic C₁₋₁₈ alkyl groups, preferably C₁₋₆ alkyl groups such as methyl, ethyl, n-propyl, iso-propyl, cyclopropyl, allyl, n-butyl, sec-butyl, tert-butyl, cyclobutyl, 2- and 3-butenyl, n-pentyl sec-pentyl, neo-pentyl, cyclopropyl, 2-, 3- and 4 pentenyl, n-hexyl, cyclohexyl, etc. The substituents include C₁₋₁₈ alkyl, C₁₋₁₈ alkoxy, C₁₋₁₈ acyl, C₁₋₁₈ acyloxy, C₁₋₁₈ acylamino, C₁₋₁₈ alkyloxycarbonyl, halogen (fluorine, chlorine, bromine and iodine), nitro, hydroxy, amino, mono- and di-(C₁₋₁₈ alkyl)amino, carboxy, carboxamide, phenyl, phenoxy, phenylamino, benzoyl, etc. The C₁₋₁₈ moiety of the substituents includes the alkyl groups mentioned herein before.

Aryl groups (hereinafter also referred to as "Ar"-groups) within the meaning of the invention include unsubstituted and substituted C₆₋₁₄ aryls and heteroaryls (the latter containing 1 to 3 heteroatoms independently selected from N, O and S) such as phenyl, naphthyl, anthracenyl, indenyl, pyridyl, chinolinyl, carbazolyl, purinyl, etc. The substituents of the aryl and the heteroaryl groups are those mentioned for the alkyl group above.

Organosilyl groups within the meaning of the invention include triorganosilyl groups such as trialkylsilyl groups and triarylsilyl groups wherein the alkyl and aryl groups may be same or different and are alkyl and aryl groups as defined above. Also contemplated are triorganosilyl groups having mixed alkyl and aryl groups. Other members of organosilyl groups are higher siloxy-alkyl groups such as Si[-0-(CH₂)ₙ-E]₃, Si[-(CH₂)ₙ-E]₃ and C[-(CH₂)ₙ-E]₃, wherein E is OH, NH₂, NHAlk, N(Alk)₂, SH, SAlk, SiAr₃, SiAlkAr₂, Si(Alk)₂Ar, Si(OAr)₃, Si(OAlk)Ar₂ or Si(OAlk)₂Ar (wherein R is an alkyl group as defined above and Ar is an aryl group as defined above) and n is an integer from 2 to 5. Particularly preferred are tri-C₁₋₆ alkylsilyl groups including trimethylsilyl, triethylsilyl, triisopropylsilyl and tri-t-butylsilyl groups.

Organoboryl groups within the meaning of the invention include dialkyboryl and diarylboryl groups, wherein the alkyl and aryl moieties are as defined above.

The organotin groups, organoalane groups, organozinc groups, organogermane groups, organogallane groups and organophosphorous groups within the meaning of the invention carry alkyl, aryl and heteroaryl groups as defined above.

The metal ions include alkali metal ions such as lithium, sodium and potassium ions, and earth alkaline metal ions such as magnesium and calcium ions.

The transition metal complexes include complex ligands containing Ti, Cr, Mn, Fe, W, Co, Ni, Rh, Ru, and Lanthanides like Eu, Nd etc.

The residues R_{a'}-R_{h'} are - at each occurrence, independently selected from H, halogen, nitro, hydroxyl, an alkyl group, an aryl group, an alkyloxy group an aryloxy group, an alkylcarbonyl group, an arylcarbonyl group, a mercaptoalkyl group, a mercaptoaryl group, a carboxy group, a carbamide group, an alkyloxycarbonyl group, a thioester group, a vinylidene group, and an arylidene group, meaning that those groups can be same or different, even if - as in case of R_{a'}, R_{b'} etc. - they are occurring more than once. The alkyl and aryl groups in the definition of R_{a'}-R_{h'}, are unsubstituted or substituted, saturated or unsaturated alkyl (Alk) or unsubstituted or substituted aryl (Ar) groups as defined herein before, respectively. Said alkyl and aryl groups may also be fused to the aromatic core structure at more than one position. The vinylidene and arylidene groups include the above mentioned C₁₋₁₈ alkyl and C₆₋₁₄ aryl groups (the latter including homo- and heteroaryls) that are bound to the aromatic core structure via a double bond. Preferred residues R_{a'} - R_{h'} are H, alkyl groups including unsubstituted and substituted, saturated and unsaturated C₁₋₁₈ alkyl groups, or aryl groups including unsubstituted and substituted C₆₋₁₄ aryls and heteroaryls.

Preferred compounds of aspect (1) of the invention are those compounds of formulas (I) to (IV), wherein n is 0 to 5 and the residues R₁ - R₄ are independently selected from organosilyl groups, organoboryl groups, metal ions alkyl groups and aryl groups. Particularly preferred are those compounds, wherein n is 0, 1 or 2. Furthermore preferred are compounds wherein the residues R₁ - R₄ are organosilyl groups selected from tri-C₁₋₆alkylsilyl groups including trimethylsilyl, triethylsilyl, triisopropylsilyl and tri-t-butylsilyl groups; organoboryl groups selected from dialkyboryl and diarylboryl groups; metal ions including alkali metal ions such as lithium, sodium and potassium ions, and earth alkaline metal ions such as magnesium and calcium ions; alkyl groups selected from unsubstituted and substituted, saturated and unsaturated C₁₋₆ alkyl groups, or aryl groups selected from unsubstituted and substituted C₆₋₁₄ aryls and hetreroaryls. Furthermore preferred are compounds wherein R_{a'} - R_{h'} are H, alkyl groups including unsubstituted and substituted, saturated and unsaturated C₁₋₁₈ alkyl groups, or aryl groups including unsubstituted and substituted C₆₋₁₄ aryls and heteroaryls.

In another particularly preferred embodiment the compound has the structure (Ia) 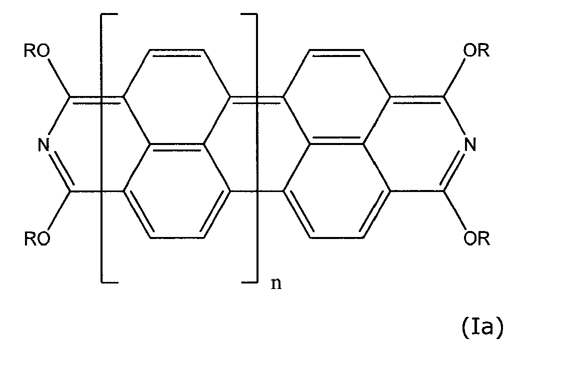 wherein n has the meaning as defined hereinbefore and R has the meaning as R₁ - R₄ defined hereinbefore. In this embodiment it is specifically preferred that n is 0 or 1 and R are trimethylsilyl groups.

In aspect (3) of the invention refers to a method for preparing the pyrene- and rylene-type compound of formulas (I) to (IV) as defined above, which comprises
(a) reacting starting compounds of the formulas (I) to (IV), wherein R₁ - R₄ are H, or a tautomer thereof, with a strong, alkali metal-containing base under reducing conditions, which can be achieved by the alkali metal-containing base itself or by providing a separate reducing agent that provides for the reduction of the system like an alkali metal, naphthalene-Na, graphite intercalation compounds like C6K, C8K, (or graphite intercalation compounds also containing lithium or sodium), electrochemistry, etc., and,
(b) if R is other than a metal, reacting the product of step (a) with a minimum of appropriate equivalents of a silylating, borylating, alkylating, arylating, organotinylating, organoalanylating, organozincylatating, organogermanylating, organogallanylating, organophosphorylating and transition metal complex-forming agent.

According to the invention it is preferred that the reducing agent and the strong base are selected from alkali metals such as lithium, sodium and potassium, in combination with alkali metal amides (MNR₂, wherein M is an alkali metal, R is independently selected from H, alkyl, trialkylsilyl, and aryl as defined above, notably is LiNH₂, NaNH₂, etc.), alkali alcoholates (such as RONa, ROK, etc. (wherein R is C₁₋₆ alkyl as defined above), metal hydrides and complex metal hydrides (such as LiH, NaH, KH, LiAlH₄, NaAlH₄, LiBH₄, NaBH₄, N(C₁₋₆ alkyl)₄ BH₄, etc.), alkyl Li (such butyl lithium, etc.), carbanionic bases or mixtures thereof.

The silylating, borylating, alkylating, arylating, organotinylating, organoalanylating, organozincylatating, organogermanylating, organogallanylating, organophosphorylating and transition metal complex- forming agent corresponds to the groups to be introduced, which carries an appropriate leaving group for coupling to the agent to the starting compound of formulas (I) to (IV) as defined above. Thus, the silylating agent is preferably a trimethylsilylhalogenide, triethylsilylhalogenide, triiso-propylhalogenide or tri-t-butylsilylhalogenide (wherein the halogenide may be a chloride, bromide or iodide), and the borylating agent is preferably a dialkylborylhalide or diarylborylhalide.

The reactions (a) and (b) may be performed in an inert aprotic solvent, such as ethers or a mixture of such solvents, or, in case the base is an alkali metal amide, an amine solvent corresponding to the base or a mixture of solvents. Further it is preferred that the reactions are performed at a temperature range of -78°C to ambient temperature; and even with heating under reflux of the solvent, or heating in an autoclave.

The validity of this concept could be verified by a reaction allowing a similar SET mechanism to form the heteroaromatic ring systems in liquid ammonia by treatment with alkali metals and amides. For both NTCDI and PTCDI, the same products as by treatment with organyllithium could be obtained as verified by NMR-spectra and chemical analysis, thus proving the above mentioned concept and reaction mechanism. These reactions can be applied e.g. to prepare nitrogen- or oxygen- functionalized compounds or related compounds. By this method, a new reaction pathway is opened to manifold of new compounds with the corresponding rylenoid polycyclic heteroaromatic ring systems, and the limitations of these reactions have to be explored. A new type of functionalization induced by various reagents is shown, leading in a straightforward manner to completely polycyclic hereroaromatic systems functionalized at the oxygen atoms of the carbonylic positions as well as the imide nitrogen atom, if present. It could be demonstrated that these reactions involve a deprotonation reaction in the case of imides as well as a SET mechanism, which can be induced by different means like reaction of the imides with an alkali metal amide/alkali metal solution in liquid ammonia or even by reactions with organyl lithium compounds. The reaction sequence presented here was hitherto unknown.

Two new compounds, 1,6-Diaza-2,5,7,10-tetrakis(trimethylsilyloxy)-pyrene (1) and 1,8-Diaza-2,7,9,14- tetrakis(trimethylsilyloxy)-peroperylene (2) could be prepared by different reaction sequences thus prooving the concept and were fully characterised by multinuclear NMR spectroscopy, UV/VIS spectroscopy and single crystal x-ray diffraction. Both compounds can be considered as modified rylene systems with silylated hydroxy groups allowing further functionalizations.

The absorption maxima of the UV/Vis spectra of compound (1) in CH₂Cl₂ are centered at 321, 340, 356, 374, 395, 418, 445 nm, those of compound (2) are at 293, 305, 402, 426, 445, 487 nm.

The method of synthesis has been tested successfully for different systems. The properties and structures of the newly accessible systems are shown exemplarily (e.g. n = 1 or 2, R= Me₃Si).

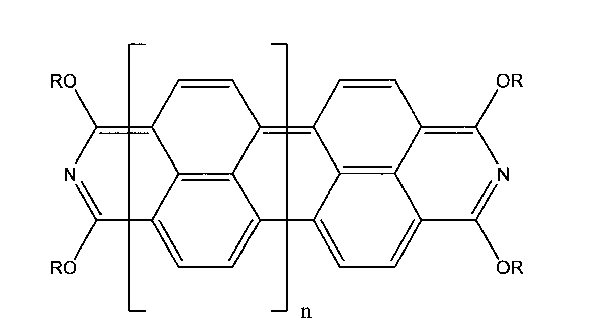

Functionalizations involving silyl- or boryl- groups as well as any kind of electrophiles are feasible.

Up to now, syntheses of rylene-type or related heteroaromatic systems have only allowed functionalizations of the amide function, substitutions at the core structure or a reduction of the carbonyl groups by diminuation of the aromatic system (P.J. Stang et al., J. Am. Chem. Soc. 117, 6273-6283 (1995)). All properties of these rylene-type systems (respectively structurally analogs compounds) accessible so far are based upon these aspects of synthesis. Conventional reduction methods only permit a complete hydrogenation of the carbonyl function, and the conjugated n-system responsible for many optical and electronic effects will not be retained in manner that it can interact with the reduced imide system, like it is the case if the carbonyl groups have been fully hydrogenated.

Regarding the functionalization of rylene based dyes, currently only methods are described in the literature allowing a derivatization either at the nitrogen atoms of the imide group by various ligands or by functionalization of the aromatic core structure (e.g. H. Langhals, Helvetica Chimica Acta 88, 1309-1343 (2005)). By these methods, fluorescent dyes are accessible allowing a tuning of the absorption and emission wave lengths. The presented new method offers an access to a new type of heteroaromatic rylenoid type dyes, in which the functional heteroatom group has been electronically and chemically modified (therefore, these systems are named MORYLENES). The accessible structural features displayed in Scheme 2 have not been described in literature before and is currently not accessible by other types of syntheses in these aromatic systems.

The syntheses and route described here are not known as a possibility for the derivatization of specifically substituted heteroaromatic systems, e.g. pyrene and peroperylene-type compounds or any of classes of compounds mentioned before. Furthermore, neither methods of synthesis deriving from the corresponding basic structures, nor the compound types described in the following sections have been reported so far. The following scheme exemplifies the possibilities for the functionalization of structural units such as imides or carbon anhydrides, now accessible through the new method, which are linked once or several times to an aromatic systems in the 1,2 and/or 1,3 position (see Scheme 2) or which are present in the educt in a different form.

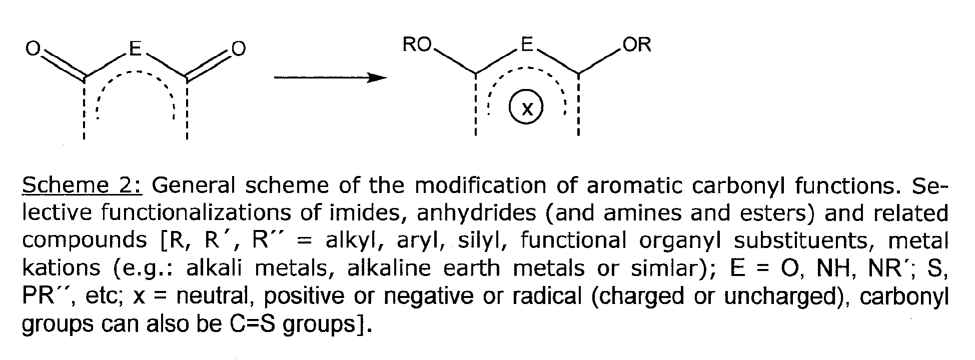

Contrary to the new methods of syntheses described here, the preparative routes known so far allow only the access to the basic structure of aza- substituted rylene type aromatics in poor to moderate yields (e.g. about 40% for 2,7 diazapyrene as well as 6% for 2,9- Diazadibenzo[cd,lm]perylene; "peroperylene") (P.J. Stang et al., J. Am. Chem. Soc. 117, 6273-6283 (1995)).

A selective reaction leading to the basic structures or of their derivates to the substances described in Scheme 2 has been not reported so far, since the compound class are only accessible by the method reported here and have not been documented so far in the literature. The fact that the reported class of compounds are suitable as fluorescence active material and/or dyestuffs is proven by the representative measurements of the corresponding parent compounds on the basic structure and on related systems (U. Wenzel, H. G. Löhmannsröben, J. Photochem. Photobiol. A 96, 13-18 (1996) and H. Langhals, W. Jona, Angew. Chem. 110, 998-1001(1998)).

So far there have been no descriptions which would enable a functionalization of the keto-groups in rylene-type systems (and related compounds). The new method of syntheses allows for example an easy access to the following important and so far unknown systems (R= alkyl, aryl, silyl, functional organyl substituents, metal kations (e.g. alkali metals, alkaline earth metals and the like), and the structures and spectroscopic properties of 1,6-Diaza-2,5,7,10-tetrakis(trimethylsilyloxy)-pyrene (1) and 1,8-Diaza-2,7,9,14- tetrakis(trimethylsilyloxy)-peroperylene (2) are described here.

Organolithium compounds may not only act as deprotonating agents, but also can act as electron donors in electron transfer reactions (D. W. Brown et al., J. Chem. Soc., Perkin Trans. 1, 2337-43 (1997) and T. Hattori et al., Tetrahedron Lett. 36, 4821-4824 (1995)). This is also the case in the imides of heteroaroaromatic rylene systems, thus leading to a new electron rich aromatic heterocycles and being derivatives of the enhanced aromatic heterocyclic ring system (e.g. benzoperylenes). It is important to note that the organometallic base added as a deprotonating agent also serves as an electron donor enabling SET reactions to form the anionic intermediate of the above mentioned ring systems. The kinetics of the imide deprotonation as well as electron transfer reaction are faster than the deprotonation of the aromatic core fragment. Such type of reaction is unprecedented in this context and also not common in the case of organolithium chemistry. A schematic representation of the organolithium route (cf. experimental section route A) is depicted in Scheme 3.

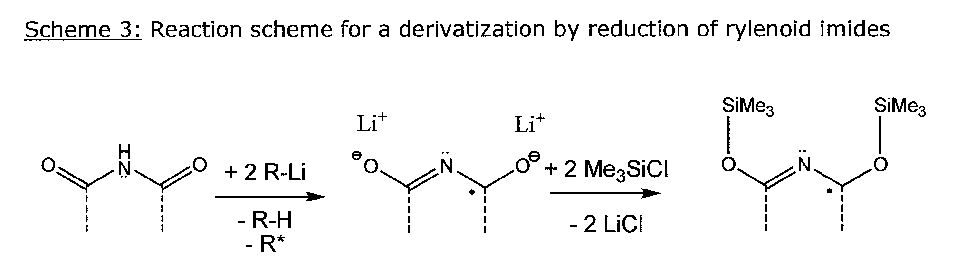

Thus, per imide group, two equivalents of organyl lithium reagent are consumed. If an even number of imide groups is present within the heteroaromatic system, the resulting heteroaromatic structure will show an even number of p-electrons within the aromatic system.

If the latter procedure is successful, the same reaction should be feasible by treatment of the imides with an alkali metal amide (e.g. LiNH₂, NaNH₂) in liquid ammonia and the presence of an alkali metal as electron transfer system according to Scheme 4 as a prove the validity of the concept. This route is describes in the experimental section as route B.

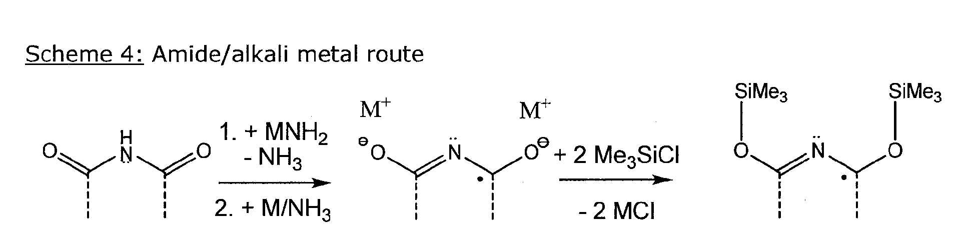

For these reasons, both routes were applied to NTCDI and PTCDI to study the deprotonation and sylilation in detail.

When the organyl- lithium route (see examples) was applied to NTCDI and PTCDI, in both cases single crystals of the tetrasilyloxy substituted heteroaromatic diazapyrene (1) and diazaperoperylene (2) could be achieved and are described in the following section.

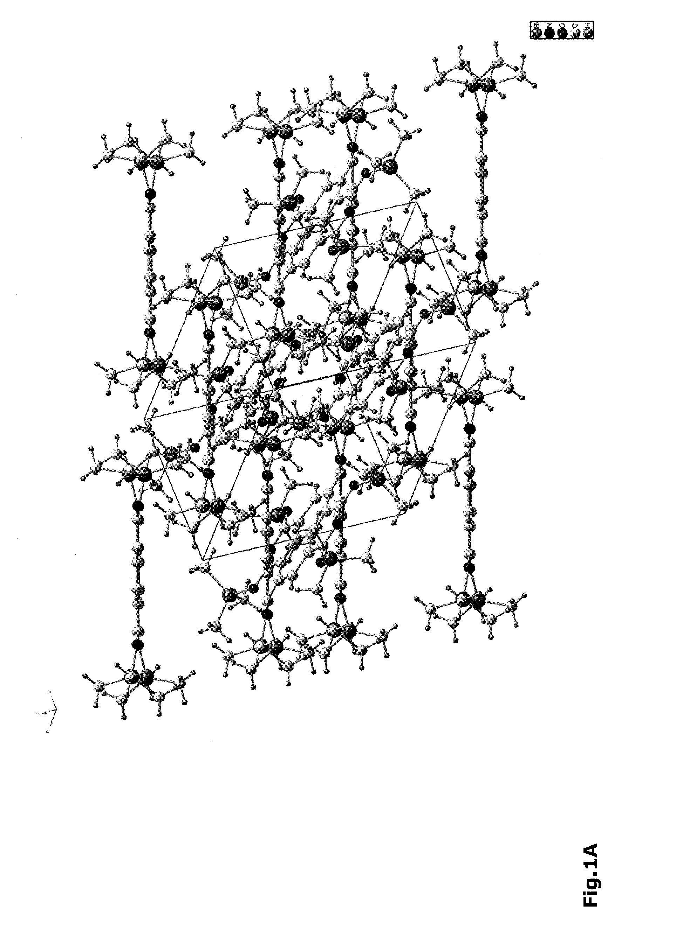

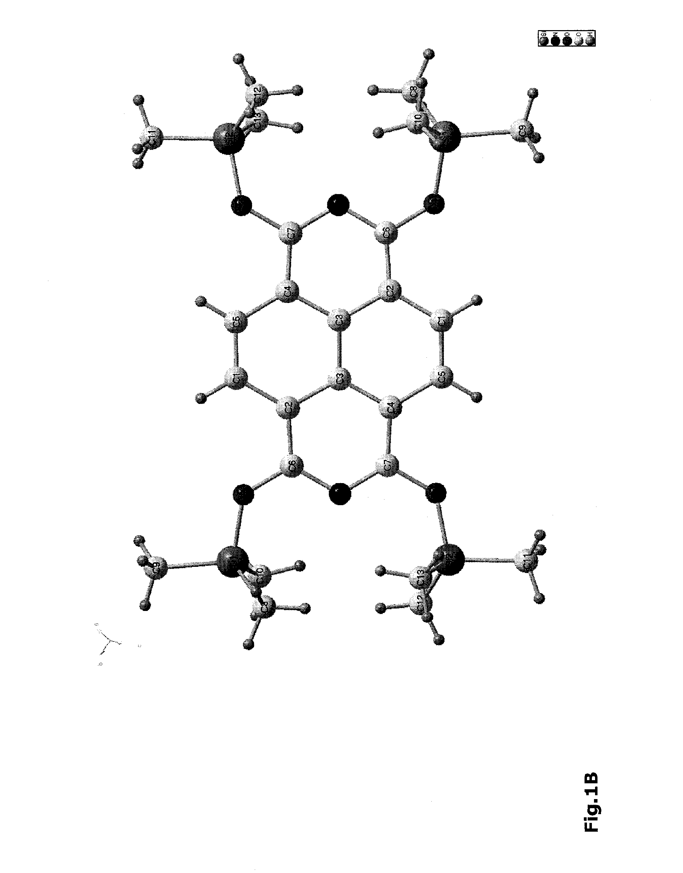

Bright yellow single crystals suitable for x- ray structure determination of (1) were obtained from methylenechloride/THF from the organyl lithium route. (1) crystallizes in the monoclinic system, space group P2(1)/n with four formula molecules per unit cell [a = 11.2691(4) Å, b = 11.1723(4) Å, c = 14.0434(5) Å, β = 113.346(2)°, V = 1623.33(10) Å³, Z = 4, Final R indices [I>2sigma(I)] R1 = 0.0398, wR2 = 0.1099, R indices (all data) R1 = 0.0536, wR2 = 0.1211]. The packing diagram and the molecular structure of (1) as well as selected bond length and angles are depicted in Fig. 1.

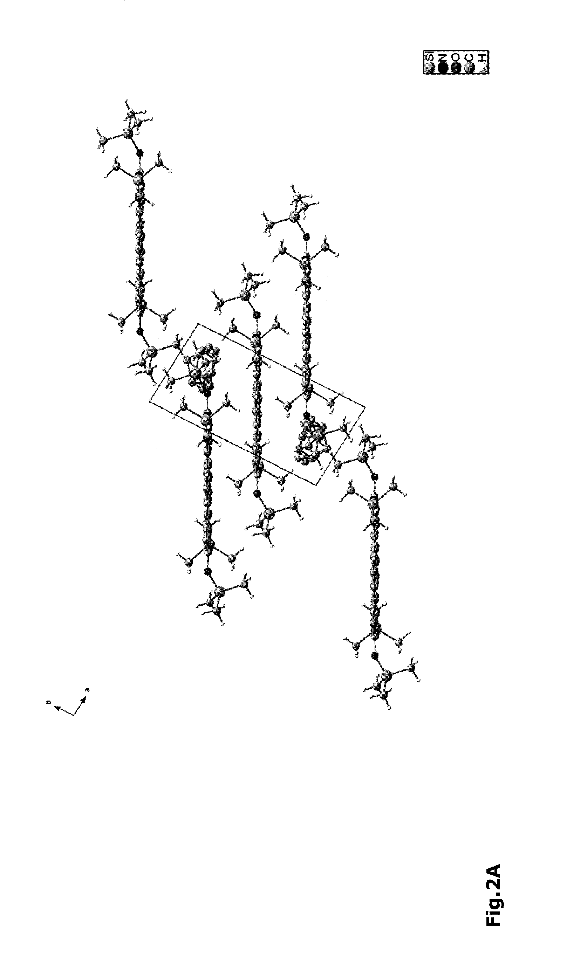

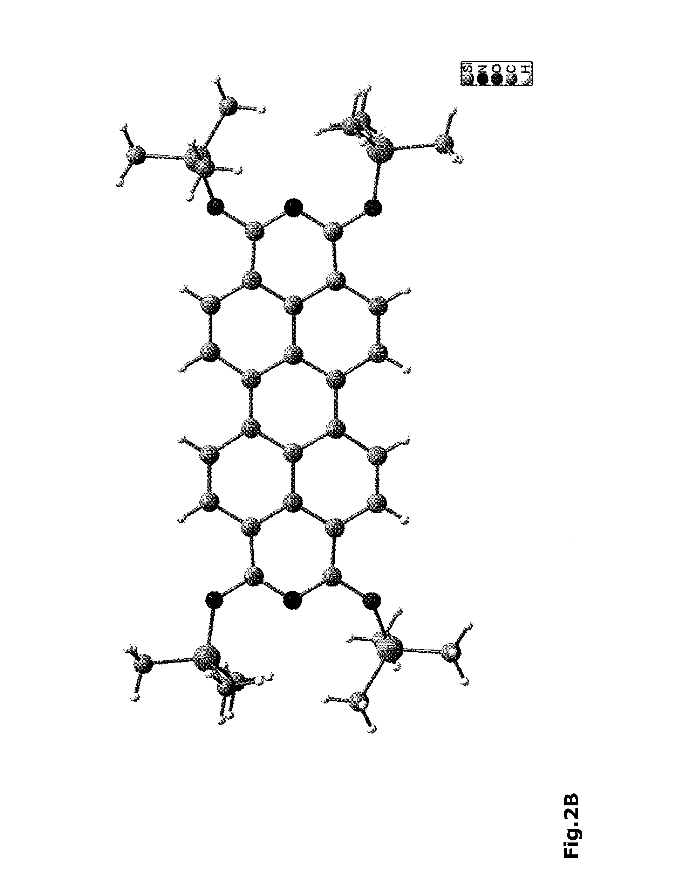

Bright orange single crystals of compound (2) suitable for x-ray structure determination were obtained from a methylenechloride/THF solution from the organyl lithium route. (2) crystallizes in the triclinic system, space group P-1, with one formula unit per unit cell [a = 6.2135(6) Å, b = 13.4802(13) Å, c = 13.7305(15) Å, α = 103.292(5)°, β = 93.435(5)°, γ = 98.956(6)°, V = 1100.11(19) Å³, Z = 1, final R indices [I>2sigma(I)] R1 = 0.0652, wR2 = 0.2166, R indices (all data) R1 = 0.0743, wR2 = 0.2266]. Some solvent is incorporated in the crystal structure, which could not be refined properly. The packing diagram and the molecular structure of (2) and selected bond length and angles are depicted in Fig. 2.

The molecular structures of the naphthalene type system (1) and the perylene type system (2) reveal some striking features. (1) and (2) show completely planar aromatic hetereocyclic ring systems where the carbonyl groups are converted to silyloxy - groups in α- position to the nitrogen heteroatom. This is an absolutely new kind of derivatization of rylene type compounds unprecedented so far and therefore enables a new access to a selective functionalization and conversion of imide type ligands into hydroxyl derivatives of those aforementioned compounds. Both systems reveal a fully planar carbon network, and no hydrogen atoms are present at the nitrogen atoms. Interestingly, both the imide derivatives reveals a bent C-O-Si group with all silicon atoms directed towards the apical nitrogen atoms, although this conformation is sterically more demanding.

Both systems clearly indicate that the former imide type carbonyl groups are converted to fully silylated C-O- functions, with the trimethylsilyloxy groups at both ends of the molecules directed in a sterically demanding maner towards the nitrogen atom of the heteroaromatic core structure. The structures clearly proove that not only a deprotonation of the imide proton has been achieved, but also an electron transfer must have taken place to allow the formation of the new electron rich heteroaromatic pyrene and peropyrene derivatives (1) and (2). The solid state structures of (1) and (2) also reveal, that all silyl groups are arranged in a sterically demanding manner and are directed towards the nitrogen atoms of the heteroaromatic system.

The NMR spectra of the isolated crystals of tetrasilyloxy substituted diazapyrene (1) in CD₂Cl₂ reveal the following data: The ²⁹Si spectrum displays one signal at 23.27 ppm (¹J[²⁹Si¹³C] = 59.6 Hz). The ¹H NMR spectrum exhibits two signals at 7.80 ppm (4 H) and 0.50 ppm (36 H), and in the ¹³C NMR spectrum five resonances can be detected (one for the trimethylsilyl groups at 0.40 ppm and four signals in the aromatic regions at 153.31 ppm (C-O-Si), 133.41 (w), 119.54 (s) and 111,68 (s) ppm. These data clearly indicate a symmetry in solution of the silylated carbon framework which is close to D₂ₕ and in full accordance with the single crystal x-ray structure determination shown in figures 1 and 2.

The NMR spectra of the isolated crystals of the tertrasilylated peropyrene 2 reveal the following signals in CDCl₃: The ²⁹Si NMR signal shows one resonance at 21.21 ppm clearly indicating a C-O-Si-group. The ¹³C NMR spectrum exhibits a signal at 1.09 ppm for the trimethylsilyl groups (¹J[¹³C²⁹Si] = 65.5 Hz) and seven signals in the aromatic region at 154.80 ppm (C-O-Si) 134.61 (w), 126.68 (s), 124.51 (w), 122.74 (s), 120.23 (s), 110.99 (s) which can all be attributed to the planar and highly symmetric perylene type carbon framework. The ¹H NMR spectrum reveals two duplettes centered at 8.70 ppm and 8.31 ppm (intensities 4 H each and one signal at 0.56 ppm (36H)). These NMR data fully correspond with the structure of the compound in the solid state which reveals a symmetry of the carbon atom framework close to D₂ₕ.

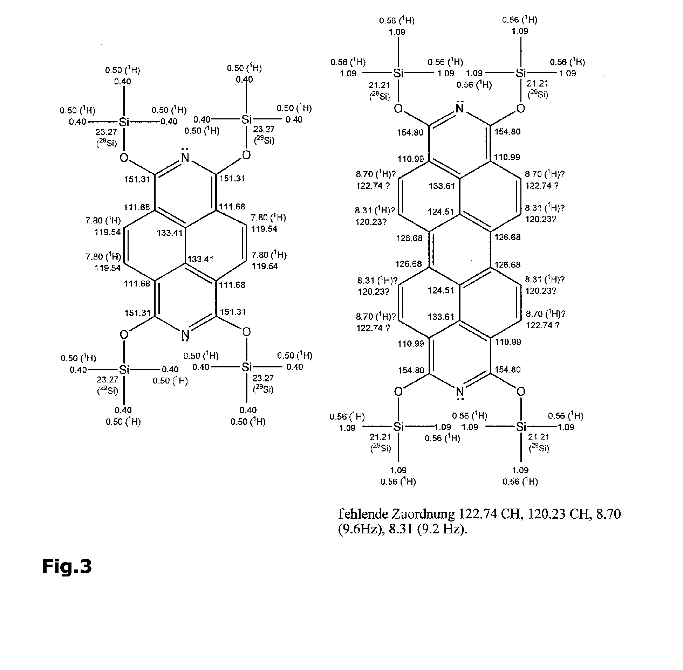

An attribution of the NMR data to the structural framework is depicted in Fig. 3 and is based upon the intensities and positions of the relevant signals, which makes the attribution evident for (1), but the assignment of the ¹H and ¹³C NMR data only for the aromatic CH signals of (2) have yet to be confirmed.

The ²⁹Si NMR spectra reveal for both substances (1) and (2) chemical shifts in the range of δ = 20 ppm, clearly indicating a formation of Si-O- bonds and not a silylation of the nitrogen atoms or the aromatic core structure. Although in principle, a 1,3 - silyl shift would be feasible leading to a mixture of O-Si- and N-Si-isomeres, only signals for the oxygen silylated compounds are detectable. In principle, an N,O- 1,3- silyl group migration would be feasible according to Scheme 5, the ²⁹Si- NMR data of 1 (δ = 23.27 ppm) and 2 (δ = 21.21 ppm) as well as the corresponding solid state structures indicate the formation of the O,O'- disylilated products for (1) and (2), an equilibrium of the N,O- and O,O '- disylilated species in solution could not be detected. This reveals a higher stability for the silyloxy derivates. The analogs derivatives of silylated chinoline isomers also only reveals the formation of the O- silylated products (H. Sachdev et al., to be published in 2010).

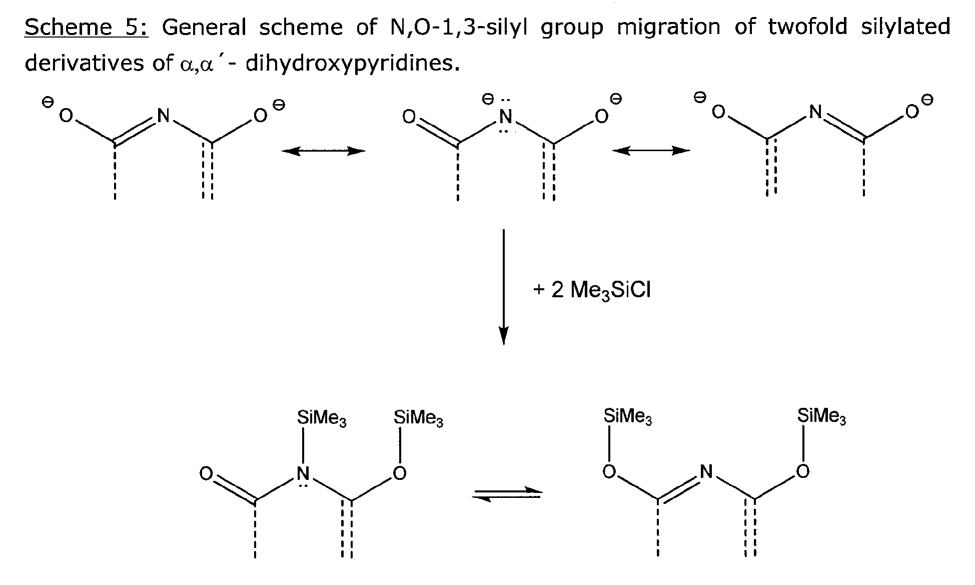

A comparison of the key structural features and spectroscopic properties of (1) and (2) clearly demonstrates the strong chemical relationship between these new type of heteroaromatic pyrene and peropyrene derivatives, as indicated e.g. by the similar C-N-, C-O- and Si-O- distances and the corresponding C-N-C- angles given in table 1. The strong fluorescence of these compounds is already obvious in solution as well as in solid state, under daylight conditions as well as by irradiation with UV and is documented by UV/VIS spectroscopy.

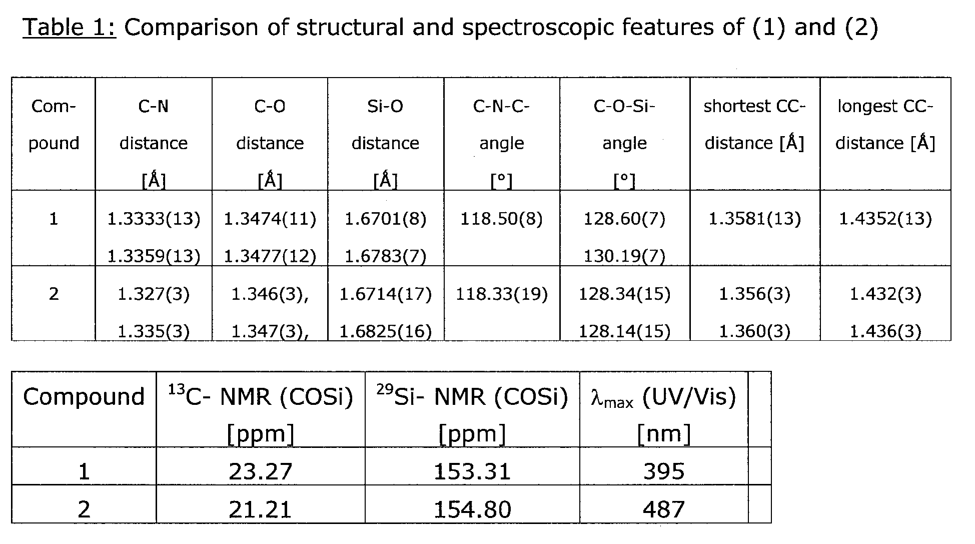

The data depicted in Tab. 1 clearly demonstrate the structural relationship of (1) and (2) and their difference compared to the related imides. The structural data of imides of NTCDI and PTCDI derivates are rather similar and reveal in general the following data for the bond length (in [Ǻ]: C=O ~1.22, C-N ~1.38-1.40, CT_{N-(C=O)-C} 1.48) (L. Feiler et al., Liebigs Ann., 1229-1244 (1995)). Therefore, the exocyclic CO- bond length in the systems (1) and (2) is enlarged by approx. 0.12 Ǻ indicating the formation of C-O single bonds, whereas corresponding CN- bonds of the newly formed heteroaromatic ring system are shortened by approx. 0.06 Å indicating an increase of bond order.

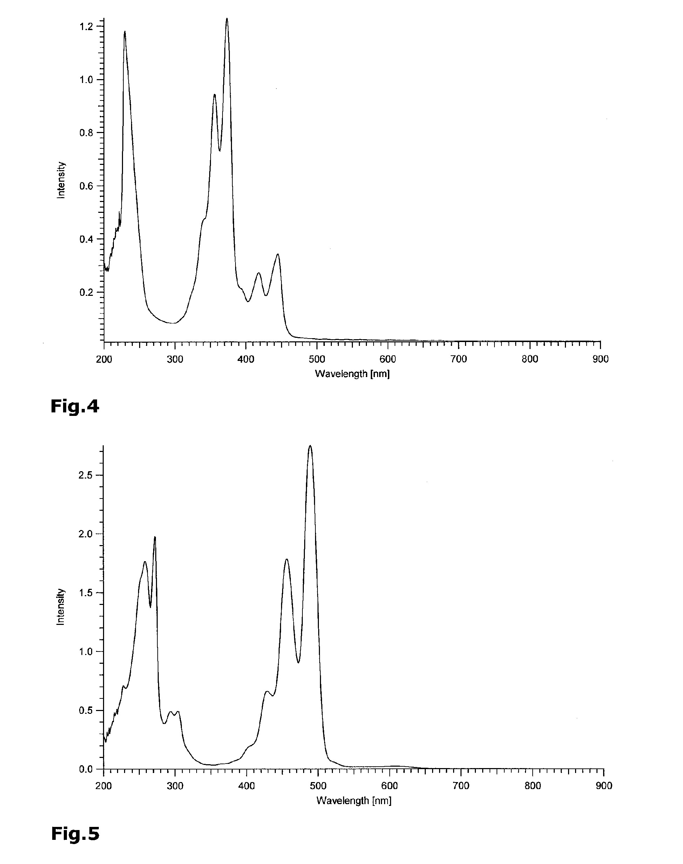

The UV/VIS absorption spectra of compound 1 and 2 are depicted in Figs. 4 and 5. Absorption maxima of 1 are detected at the following wavelengths [nm]: λ = 321, 340, 356, 374, 395, 418, 445; the absorption maxima of 2 are observed at the wavelengths [nm]: λ = 293, 305, 402, 426, 454, 487. The UV/Vis spectra for compound 1 and 2 reveal a strong shift of the absorption maxima to the lower wavelength region compared to similar perylene or naphtalene type systems (S. Müller, K. Müllen, Philos. Trans. R. Soc., A 365, 1453-1472 (2007)).

Absorption maxima are detected at the following wavelength for Compound 1, solvent CH₂Cl₂ [nm]; λ = 321, 340, 356, 374, 395, 418, 445., Absorption maxima: λ₁ = 356 nm, λ₂ = 374 nm, λ₃ = 418 nm, λ₄ = 445 nm; Emission maxima: λ₁ = 454 nm, λ₂ = 481 nm, λ₃ = 515 nm; Excitation maxima: λ₁ = 358 nm, λ₂ = 375 nm, λ₃ = 419 nm, λ₄ = 445 nm

Absorption maxima are detected at the following wavelength for Compound 2, solvent CH₂Cl₂ [nm]: λ = 293, 305, 402, 426, 454, 487., Absorption maxima: λ₁ = 430 nm, λ₂ = 456 nm, λ₃ = 490 nm; Emission maxima: λ₁ = 285 nm, λ₂ = 340 nm, λ₃ = 356 nm, (λ₄ = 369 nm), λ₅ = 503 nm, λ₆ = 540 nm, λ₇ = 577 nm; Excitation maxima: λ₁ = 430 nm,; λ₂ = 457 nm, λ₃ = 492 nm

Due to these findings, the functionalization of the corresponding imides proceeds via a deprotonation and an electron transfer reaction leading to the observed structures depicted in Scheme 6 of (1) and (2). The first step (no.1) of this route can be accomplished in principle by either treating the corresponding imides with a solution of an alkali metal amide (e.g. LiNH₂, NaNH₂) and an alkali metal (e.g. Li, Na) in liquid ammonia or by reaction of an organyl lithium reagent acting as deprotonating as well as electron transfer reagent like e.g. butyl lithium.

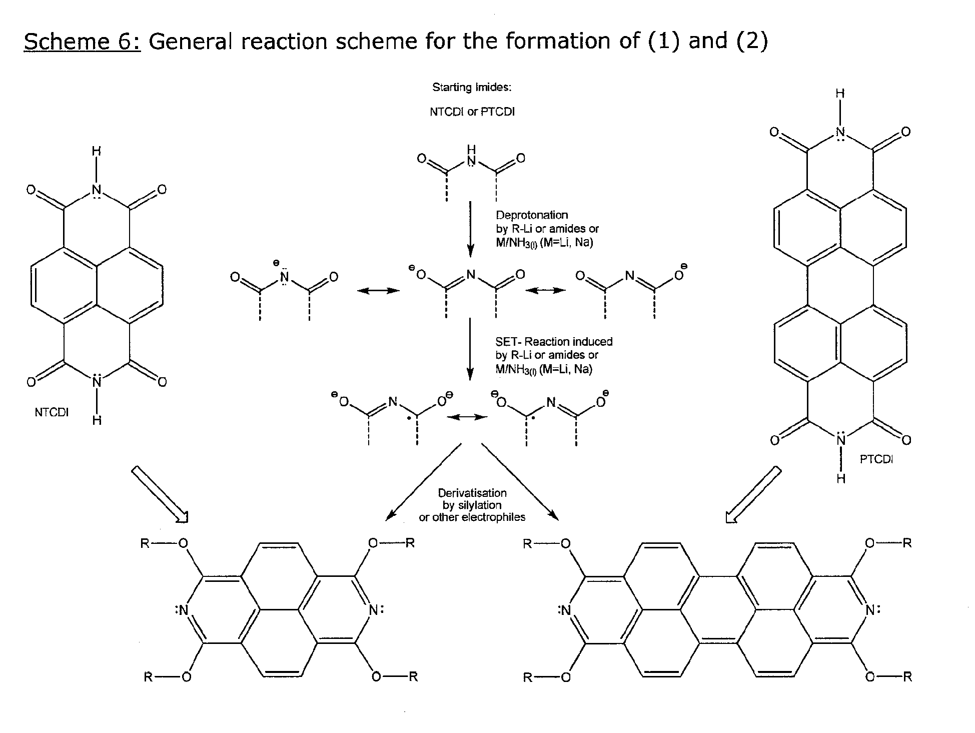

It is interesting that by the reaction of organolithium reagents, not only a deprotonation occurs but an SET reaction is observed as well and thus more equivalents of the starting reagent are consumed. In both cases the same products could be obtained, clearly proving the above mentioned mechanism.

The validity of this concept could be verified by a reaction allowing a similar SET mechanism to form the heteroaromatic ring systems in liquid ammonia by treatment with alkali metals. For both NTCDI and PTCDI, the same products could be obtained as verified by NMR-spectra and chemical analysis, thus proving the above mentioned reaction mechanism. The described reaction mechanism allows a functionalization of electron deficient imide groups to α,α' - dihydroxypyridin derivatives and therefore opens a new perspective in aromatic chemistry. The strong UV fluorescence of these compounds allows the use of these molecules as a new type of dyes similar to rylene compounds. The obtained products can also be used as building blocks for self-organised systems because of the presence of two nitrogen functional sites within the modified rylene type molecules.

Both compounds (1) and (2) are highly soluble in nonprotic solvents like alkanes, arenes, halogenated hydrocarbons, ethers, ketones and esters and are to a certain extend stable in alcohols. Any traces of water lead to a pronounced hydrolysis of the system by formation of an insoluble red deposit for (1) and (2), which are assumed to be the corresponding derivatives of the α,α'- dihydroxypyridine type analogs of (1) and (2) depicted in fig. 13. The compounds (1) and (2) are sensitive to hydrolysis due to the presence of the trimethylsilyloxy groups. Hydrolysis leads to insoluble reddish deposits, presumably the hydroxy derivatives of (1) and (2). It has to be characterized whether the hydrolysis products of these systems display a α,α'-dihydroxypyridin-pyrimidon equilibrium as described in Scheme 7 for compound (1) (analog for 2).

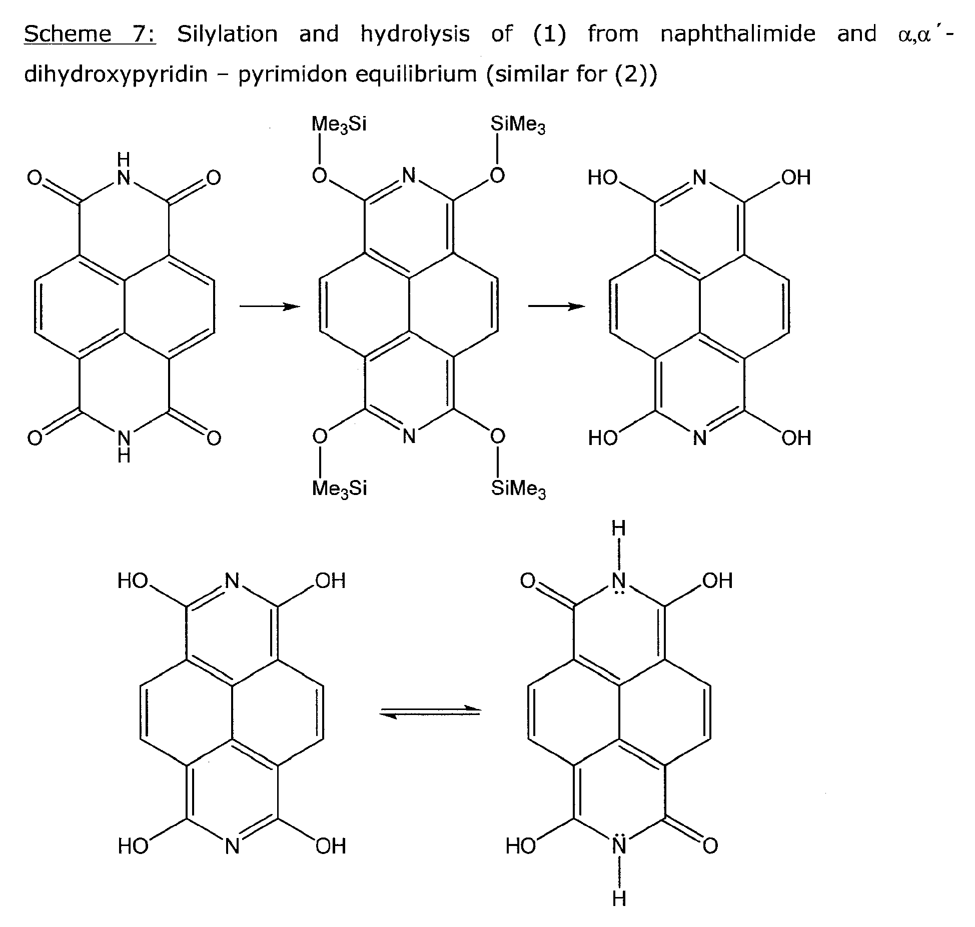

From the ionic intermediates many other functional modified rylene type compounds are now accessible which allows a tuning of the emission wave lengths of the aromatic ring system, as shown in Scheme 8.

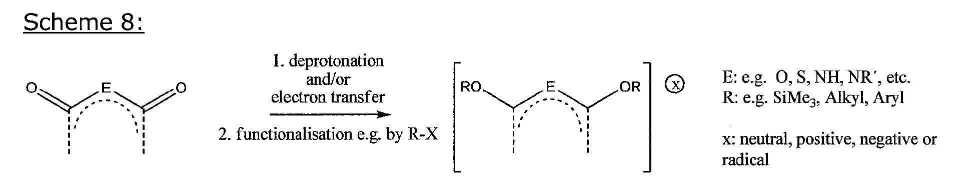

It could be shown that reagents enabling deprotonation and SET reactions result in the formation of new rylenoid systems 1 and 2 from rylene type imides. The deprotonation and electron transfer reaction can be obtained either by reaction of the imides with organyl lithium reagents or by reaction of an alkali metal amide/alkali metal solution in liquid ammonia according to Scheme 6.

The resulting intermediate salts can be further functionalized by e.g. treatment with organylelementhalides like e.g. Me₃SiCl leading to the compounds (1) and (2).

By the presented method new types of functional rylenoid systems are easily accessible in high yield, showing extremely high solubility in organic solvents and reveal strong fluorescence in the blue (1) to green (2) region depending on the size and nature the n-electron system. The presented method in general allows a selective functionalization of organic imides resulting in heteroaromatic systems richer in electrons according to Scheme 9, which are called modified rylenes (MORYLENES).

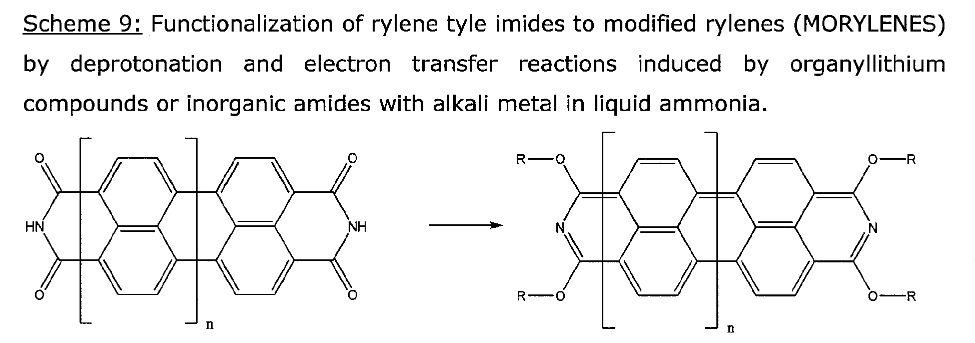

By the described methods silylations of the intermediate salts resulting from SET (and deprotonating reactions) were made leading to valuable new heteroaromatic pyrene and peroperyene systems (1) and (2), which can be e.g. further functionalised by reaction with electrophiles like organylhalogenboranes. Such reactions have been successfully carried out with compound (1) and (2). Treatment of (1) with four equivalents of a reactive boron halide (Phenyl₂BBr) in a stoichometric ratio led to the straightforward elimination of Me₃SiBr and the formation of the tetraborylated product, thus modifying the structure in an unprecedented manner. Such a borylation in general can lead to N,O- and O,O'-derivatives, and also a fourfold coordinate system might be feasible leading to switchable functional fluorescent dyes (H. Sachdev et al., to be published in 2010). From the silylated compounds (1) and (2) as well as from the salt intermediates a new route to a manifold of new compounds is facilitated by further derivatization with electrophiles, nucleophiles or by a reductive cleavage of the silyloxy groups, and derivates of the diaza homologues of aromatic pyrene and peropyrene systems may be accessible.

The systems shown in the following figure have not been documented in literature before, the corresponding synthesis of the oxa-derivates instead of the aza systems is possible following the same procedure after the first examination (H. Sachdev et al., to be published in 2010]). In general this method can be applied to functionalize amines and related structural motives and further investigations are currently performed to show to what extent this method can be applied to obtain new functionalised organic compounds and dyes (H. Sachdev et al., to be published in 2010).

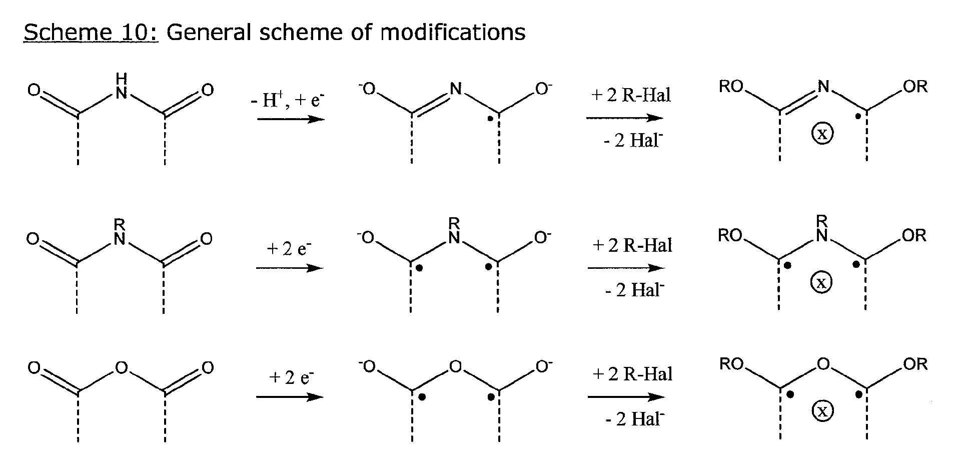

The method may be extend to anhydrides, amides and esters (aromatic and aliphatic systems) and allows the derivatization of the above mentioned newly accessible compounds. An application of these new heteroaromatic systems as fluorescence dyestuffs is feasible because of their UV/Vis spectral properties and opens new approach of these systems for optical or electronic applications. Also a linkage of the newly accessible fluorescent dyestuffs with other chromophores, use as complex ligands and building blocks for self-organized systems is feasible because of the functionality of the molecules. Due to the heteroatoms present in the polycyclic ring system (e.g. pyridine type nitrogen atoms in 1 and 2), the systems can also act as new functional molecular building blocks for applications in coordination chemistry as well as the build up of supramolecular networks. Since the compounds 1 and 2 can be considered as electron rich polycyclic aromatic compounds, the redox activity of these systems is also of interest.

The presented method is in general also applicable to other systems depicted in Schemes 10 and 11, e.g. like anhydrides and imides of five membered ring systems or systems having an odd number of imide groups (or groups previously mentioned) and first attemps also reveal silylation of the corresponding carbonyl groups (H. Sachdev et al., Synthesis and Structure of Trimethylsilyl- isochinoline, to be published in 2010).

The substitution patterns of Structures (I) to (IV) and as describes in Scheme 11 not been described in literature so far. Modifications of the aromatic core system of these new class of compounds also alows a tuning of the fluorescence properties, e.g. functionalization by donor s at the neighbouring positions to the heteroatoms (like N, O, S etc.) are accessible.

Treatment of (1) with four equivalents of a reactive boron halide (Phenyl₂BBr) in a stoichometric ratio led to the straightforward elimination of Me₃SiBr and the formation of the tetraborylated product, thus modifying the structure in an unprecedented manner. Such a borylation in general can lead to N,O- and O,O'-derivatives, and also a fourfold coordinate system might be feasible leading to switchable functional fluorescent dyes. 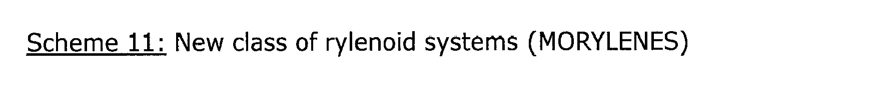 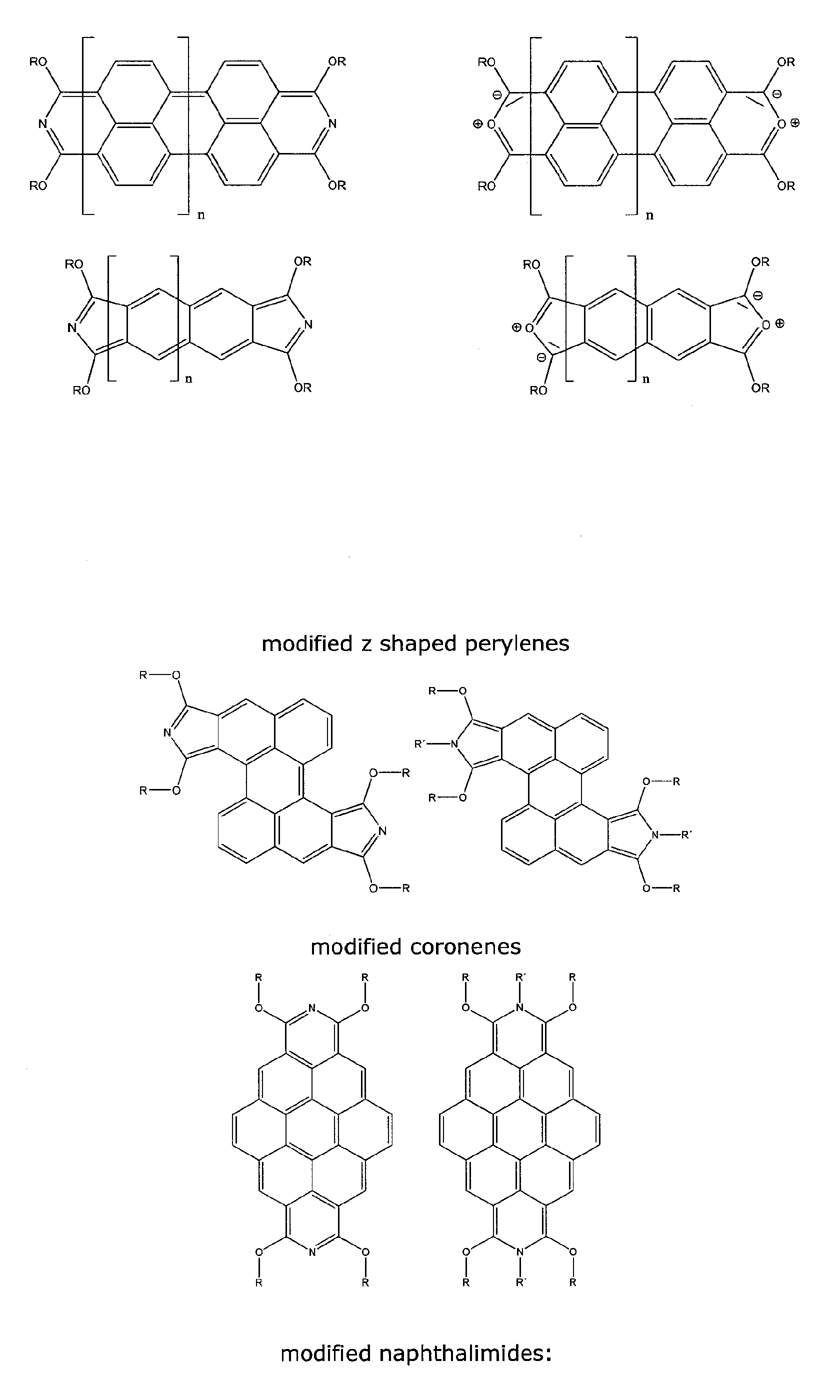 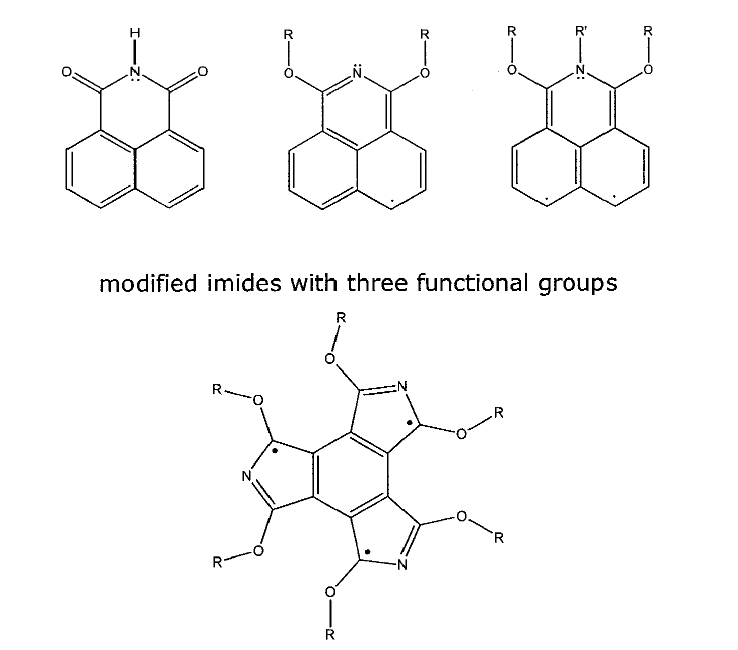 The method described here allows the synthesis of a new type of compounds with specific substitution patterns, especially of pyrene- and rylene-type aromatic systems or related heteroaromatic compounds. The newly accessible compounds can be used as synthones for further derivatizations or because of their properties (e.g. fluorescence dyestuffs, optical and electronic applications). Moreover the method is suitable for selective functionalizations of organic imides, anhydrides, amides and esters (and related groups) and for the synthesis of so far unknown substance classes derived from or showing the with the specific substitution pattern described in the following sections. The synthesis can be achieved by the reaction of the corresponding starting materials with redox- and/or redox- and deprotonating reagents (alkali metals, Grignard-reagents, metallorganyles, GIC-compounds and others, in some cases catalysts may be added) in various solvents (e.g. apolar solvents, aromatic solvents or coordinating ethers, amines or solvents or mixtures thereof) and the consequent derivatization of the intermediates with halogene functional agents (halogene organyles, organoelementhalogenides, or electrophiles in general). All of the necessary steps can be performed in a single reaction or stepwise by isolating the intermediates or by combining the approaches. Thus, the present invention provide for a new method for the synthesis for the preparation of new functional compounds (compounds of the invention) from imides, anhydrides, amides and esters (aromatic and aliphatic systems) or respectively from compounds containing an amide-, imidie-, ester or anhydride-group or compounds containing one or several of these groups is described. The synthesis of the modified pyrene- and rylene-type compounds of the invention (polycyclic aromatic compounds having one or more heteroatoms like nitrogen, oxygen, sulfur, etc. or any combination of these) is described. The compounds of the invention display one or more of the above mentioned functionalities and are accessible with the new preparation route. The derivatization of the compounds of the invention is achieved by reaction with functional organic compounds (CH-, NH-, or OH-acid systems, alcohols, amines, halides, or any compounds or ions acting as electrophiles). The compounds of the invention are heteroaromatic systems that are suitable as dyestuffs and fluorescence dyestuffs or using their fluorescent properties. The compounds of the invention or any of their derivatives are applicable for any optical or electronic applications. The compounds of the invention or any of their derivatives are applicable in electron transfer reactions. Linkage of the compounds of the invention with other dyestuffs, the use of the compounds of the invention as complex ligands, and building blocks for self-organized systems and reaction intermediates are also contemplated.

The invention is further explained in the following non-limiting examples.

### Examples

Materials and Methods: All procedures were carried out by using modified Schlenk techniques in an inert gas atmosphere. Solvents were dried by standard procedures and degassed prior to use. NTCDI and PTCDI were prepared from the corresponding tetracarbonic acid anhydrides obtained from commercial suppliers (Aldrich) according to literature methods (I. Oesterling, K. Müllen, J. Am. Chem. Soc. 2007, 129, 4595-4605) and dried intensively. Alkali metals, alkali metal amides (LiNH₂, NaNH₂), ammonia 3.0 and 1.6 m butyllithium in hexane were obtained from commercial suppliers. The compounds 1 and 2 are sensitive to hydrolysis and therefore appropriate care has to be taken.

NMR spectra were recorded with a Bruker ACP 200 S spectrometer. For ¹H- and ¹³C-NMR- spectra, C₆D₆ (δ = 7.15 ppm), CDCl₃ (δ = 7.26 ppm) and CD₂Cl₂ were used as internal standards. TMS (for ¹H-, ¹³C-, ²⁹Si-NMR), respectively, were used as an external standard. (¹H: 200 MHz; ¹³C: 55.0 MHz; ²⁹Si 39.73 MHz). Regarding the hazard potential of compounds (1) and (2), there is no specific knowledge available and the usual precautions for aromatic compounds should be taken, also by working with alkali metal amides and alkali metals in liquid ammonia. UV-VIS spectra were recorded in methylenechloride (~10 mg of sample dissolved in 5 ml or diluted appropriately) by a Perkin Elmer Lambda 35 UV-VIS spetrometer. Deviations in elemental analyes are attributed to the moisture sensitivity of 1 and 2 as well as to the formation of carbides during combustion. The data for the crystal structure of compound (1) and (2) were collected with a Bruker AXS X8 Apex CCD four-circle diffractometer with a LT cooling device (Mo-KAlpha, Lambda = 0.71073 Angstroem, graphite monochromator)and relevant information is summarized in table 2. The programs SHELXTL were used for data collection, structure solution and refinement (G. M. Sheldrick, SHELX Program for crystal structure determination, University of Göttingen, 1976-2009; G. M. Sheldrick, Acta Cryst. A 64, 112-122 (2008)). The structures of the compounds were solved by direct methods and refined by full-matrix least square methods. Anisotropic thermal parameters for all non-hydrogen atoms in the final cycles, isotropic refinement for hydrogen atoms positioned by geometry.

General method (A) according to the organyl lithium route for imides and other systems: To a slurry of approx. 2.0 g of an imide or any other type of rylenoid system in 100 ml THF or any other ether mixed with an inert solvent, 4.4 eq. of a solution of 1.6 m BuLi in hexane were added slowly at RT. A evolution of butane is observed while the mixture forms a intense coloured solution. After addition of the organyl lithium reagent, the mixture is stirred for approx. 1h and then an excess of R-Cl (trimethylchlorosilane, e.g. 6 equivalents) is added while the mixture heats up slightly. The mixture is refluxed for approx. 2h. Then all volatile material is removed in vacuum up to a level of 50%, and approx. 100 ml of methylenechloride are added. The solid residue is filtered off. From the filtrate and by extraction of the solid residue with methylenchloride, the corresponding dyes 1 and 2 can be obtained in high yield in crystalline form by cooling the solutions to 5°C and removal of the solvent until crystallisation begins. By this method, single crystals for x- ray structure determination of 1 and 2 were obtained. Approx. 50-80 % of 1 and 2 can be obtained by recrystallisation from methylenechloride.

General method (B) according to the lithium amide/ammonia route for imides: Approx. 2.0 g of an imide (NTCDI, PTCDI) are suspended in a slurry of 2.5 equivalents of an alkali metal amide in 100 ml liquid ammonia at -78°C. After the reaction mixture has reacted with the amide, (typically within 1h), 2.5 equivalents of an alkali metal (Li, Na) are added*. The suspension is stirred for approx. 2h. Then, the liquid ammonia is exchanged slowly against the same amount of an organic solvent (first toluene, then THF or any other coordinating ether) while ammonia is evaporated. At room temperature, an excess of R-Cl (trimethylchlorosilane, e.g. 6 equivalents) is added while the mixture heats up slightly. The mixture is refluxed for 3 h. Then all volatile material is removed in vacuum up to a level of 50%, and then approx. 50 ml of methylenechloride are added. The solid residue is filtered off. From the filtrate and by extraction of the solid residue with methylenchloride, the corresponding dyes 1 and 2 can be obtained in high yield in crystalline form by cooling the solutions to 5°C and removal of the solvent until crystallisation begins. This method works best with NTCDI (PTCDI has to be finely dispersed and extremely dry) and approx. 60-80 % of a crystalline fraction of 1 can be obtained by recrystallisation from methylenechloride. * Note: The amount of alkali metal (and even the alkali metal amide) is not very critical and may be larger, but then appropriate care has to be taken to ensure an excess of R-X to destroy all reactive metal and metal amide before workup with an halogenated organic solvent. E.g. for (1), Formula and molecular weight: C₂₆H₄₀N₂O₄Si₄; Mᵣ = 556.95 g/mol (NMR data are identical to route B).

| CHN analysis: | | | |
|---|---|---|---|
| Calculated [%]: | C 56.07 | H 7.24 | N 5.03 |
| Found₁ [%]: | C 55.56 | H 7.02 | N 5.09 |

### Example 1: Synthesis of 1,3,6,8-tetrakis-trimethylsilyloxy-2,7-diazapyrene (1)

1.00 g (3.8 mmol) of Naphthalintetracarboxydiimide (NTCDI) were suspensed in approximately 50 ml of THF. 10.0 ml (16.0 mmol) of a 1.6 M Butyllithium solution in hexane were added within 30 minutes.

The color of the mixture changes towards a deep purple suspension and intensifies while the mixture is kept at approximately 50°C for 30 minutes. 3.0 ml (23.5 mmol) of Chlorotrimethylsilane were added within 30 min and the suspension changed its color to a greenish to brownish tone. After the suspension is heated up to approximately 50°C, the mixture becomes yellowish. The system is heated up to reflux for approximately 1 hour. Approx. 50 % of the volatile material was removed at reduced pressure and then 100 ml of dichloromethane were added. The mixture was filtered and the residue washed with dichloromethane, toluene and heptane. A clear yellowish solution is obtained which exhibits a strong fluorescence in the blue range. By further removal of the solvent and storage at 8°C, single crystals of an intense yellow color suitable for x-ray structure determination could be obtained within a few days.

Yield of the isolated crystals: 1.02 g (48%)

Formula and molecular weight: C₂₆H₄₀N₂O₄Si₄; Mᵣ = 556.95 g/mol

| CHN analysis: | | | |
|---|---|---|---|
| Calculated [%]: | C 56.07 | H 7.24 | N 5.03 |
| Found₁ [%]: | C 55.74 | H 7.07 | N 4.68 |
| Found₂ [%]: | C 54.49 | H 6.67. | N 5.56 |

| NMR (isolated crystals in CD₂Cl₂): |
|---|
| δ²⁹Si : 23.27 |
| δ¹³C : 0.40 (SiMe₃), 111.68 (Ar), 119.54 (Ar), 133.41 (Ar), 153.31 (C-O-Si) |
| δ¹H: 0.50 (SiMe₃, 36 H); 7.80 (Ar, 4 H) |

### Selected Bond lengths [Å] and angles [°] for (1)

Si(1)-O(1) 1.6783(7), Si(2)-O(2) 1.6701(8), N(1)-C(7) 1.3333(13), N(1)-C(6) 1.3359(13), O(1)-C(6) 1.3474(11), O(2)-C(7) 1.3477(12), C(1)-C(5)#1 1.3581(13), C(1)-C(2) 1.4342(13), C(2)-C(6) 1.4056(12), C(2)-C(3) 1.4125(12), C(3)-C(4) 1.4115(12), C(3)-C(3)#1 1.4249(16), C(4)-C(7) 1.4053(12), C(4)-C(5) 1.4352(13), C(5)-C(1)#1 1.3581(13), C(7)-N(1)-C(6) 118.50(8), C(6)-O(1)-Si(1) 128.60(7), C(7)-O(2)-Si(2) 130.19(7), C(5)#1-C(1)-C(2) 120.62(8), C(6)-C(2)-C(3) 116.20(8), C(6)-C(2)-C(1) 124.02(8), C(3)-C(2)-C(1) 119.78(8), C(4)-C(3)-C(2) 120.89(7), C(4)-C(3)-C(3)#1 119.43(10), C(2)-C(3)-C(3)#1 119.68(10), C(7)-C(4)-C(3) 116.27(8), C(7)-C(4)-C(5) 123.77(8), C(3)-C(4)-C(5) 119.94(8), C(1)#1-C(5)-C(4) 120.55(9), N(1)-C(6)-O(1) 118.54(8), N(1)-C(6)-C(2) 124.05(8), O(1)-C(6)-C(2) 117.40(9), N(1)-C(7)-O(2) 118.90(8), N(1)-C(7)-C(4) 124.08(9), O(2)-C(7)-C(4) 117.02(8).

### Example 2: Synthesis of 1,10,12,21-tetrakis-trimethylsilyloxy-2,9-diazadibenzo-[cd,lm]perylene (2)

5.5 ml of a 1.6 m solution of buthyllithium in hexane (8.8 mmol) are added to a suspension of 0.80 g (2.0 mmol) of perylentetracarboxydiimide (PTCDI) in approximately 50 ml THF at room temperature. During addition, a slight rise in temperature and evolution of butane was observed. After stirring the suspension for 30 min at 50°C, 2.0 ml (15.6 mmol) of chlortrimethylsilane were added. The suspension turns bright yellow - green and also exhibits an intensive green fluorescence. The mixture is refluxed for 2 h, and then approx. 50 % of the volatile material are removed in vacuum. Approx. 100 ml of dichlormethane were added and the suspension is filtered. Needle shaped crystals of an intensive bright orange colour appeared after storage at 8°C within a few days. The crystals were isolated and washed with heptane and were suitable for single crystal x - ray diffraction structural analysis.

Yield of isolated crystals 0.79 g (57%)

Formula and molecular weight: C₃₆H₄₆N₂O₄Si₄; Mᵣ = 683.10 g/mol

| CHN analysis (deviations due to non stoichiometic incorporated THF and CH₂Cl₂): | | | |
|---|---|---|---|
| Calculated [%]: | C 63.30 | H 6.79 | N 4.10 |
| Found₁ [%]: | C 63.86 | H 8.69 | N 4.10 |
| Found₂ [%]: | C 64.31 | H 5.35 | N 4.79 |

| NMR (isolated crystals, CDCl₃): |
|---|
| δ ²⁹Si: 21.21 |
| δ ¹³C : 154.80 (Ar-C-O-Si), 134.61 (Ar), 126.68 (Ar), 124.51 (Ar), 122.74 |
| (Ar), 120.23 (Ar), 110.99 (Ar), 1.09 (SiMe₃) |
| δ ¹H : 0.56 (SiMe₃, 9 H), 8.31 (d, 9.2 Hz, 4 H, Ar); 8.70 (d, 9.6 Hz, 4 H, Ar) |

### Selected bond lengths [Å] and angles [°] for 2

Si(2)-O(2) 1.6825(16), Si(1)-O(1) 1.6714(17), N(1)-C(2) 1.327(3), N(1)-C(1) 1.335(3), O(1)-C(1) 1.347(3), O(2)-C(2) 1.346(3), C(1)-C(5) 1.409(3), C(2)-C(3) 1.417(3), C(3)-C(4) 1.420(3), C(3)-C(12) 1.421(3), C(4)-C(5) 1.414(3), C(4)-C(9) 1.432(3), C(5)-C(6) 1.417(3), C(6)-C(7) 1.360(3), C(7)-C(8) 1.436(3), C(8)-C(10) 1.421(3), C(8)-C(9) 1.422(3), C(9)-C(10)#1 1.421(3), C(10)-C(9)#1 1.421(3), C(10)-C(11) 1.445(3), C(11)-C(12)#1 1.356(3), C(12)-C(11)#1 1.356(3), C(2)-N(1)-C(1) 118.33(19), C(1)-O(1)-Si(1) 128.34(15), C(2)-O(2)-Si(2) 128.14(15), N(1)-C(1)-O(1) 118.25(19), N(1)-C(1)-C(5) 124.1(2), O(1)-C(1)-C(5) 117.7(2), N(1)-C(2)-O(2) 118.44(19), N(1)-C(2)-C(3) 124.2(2), O(2)-C(2)-C(3) 117.4(2), C(2)-C(3)-C(4) 116.7(2), C(2)-C(3)-C(12) 123.6(2), C(4)-C(3)-C(12) 119.65(19), C(5)-C(4)-C(3) 119.5(2), C(5)-C(4)-C(9) 120.4(2), C(3)-C(4)-C(9) 120.0(2), C(1)-C(5)-C(4) 117.1(2), C(1)-C(5)-C(6) 123.6(2), C(4)-C(5)-C(6) 119.2(2), C(7)-C(6)-C(5) 120.8(2), C(6)-C(7)-C(8) 121.8(2), C(10)-C(8)-C(9) 119.2(2), C(10)-C(8)-C(7) 122.3(2), C(9)-C(8)-C(7) 118.4(2), C(10)#1-C(9)-C(8) 121.35(19), C(10)#1-C(9)-C(4) 119.4(2), C(8)-C(9)-C(4) 119.2(2), C(9)#1-C(10)-C(8) 119.5(2), C(9)#1-C(10)-C(11) 118.51(19), C(8)-C(10)-C(11) 122.0(2), C(12)#1-C(11)-C(10) 121.8(2), C(11)#1-C(12)-C(3) 120.6(2)

**Table 2:Crystal data and structure refinement for (1) and (2):**

| | Compound (1) | Compound (2) |
|---|---|---|
| Empirical formula | C13 H20 N O2 Sl2 | C44 H60 N2 06 Si4 |
| Formula weight | 278.48 | 825.30 |
| Temperature | 153(2) K | 150(2) K |
| Wavelength | 0.71073 Å | 0.71073 Å |
| Crystal System | Monoclinic | Triclinic |
| Space Group | P2(1)/n | P-1 |
| Unit cell dimensions | a = 11.2691(4) Å α = 90° | a = 6.2135(6) Å α = 103.292(5)° |
| | b = 11.1723(4) Å β = 113.346(2)° | b = 13.4802(13) Å β = 93.435(5)° |
| | c = 14.0434(5) Å γ = 90° | c = 13.7305(15) Å γ = 98.956(6)° |
| Volume | 1623.33(10) Å³ | 1100.11(19) Å³ |
| Z | 4 | 1 |
| Density (calculated) | 1.139 Mg/m³ | 1.246 Mg/m³ |
| Absorption coefficient | 0.214 mm⁻¹ | 0.183 mm⁻¹ |
| F(000) | 596 | 442 |
| Crystal size | 0.41x0.33x0.20 mm³ | 1.22 x 0.40x0.08 mm³ |
| Theta range for data collection | 1.98 to 34.19° | 1.53 to 27.93°. |
| Index ranges | -16<=h<=17, -17<=k<=17, | -8<=h<=8, -17<=k<=17, |
| | -22<=l<=22 | -18<=l<=18 |
| Reflections collected | 48207 | 18799 |
| Independent reflections | 6684 [R(int) = 0.0255] | 5149 [R(int) = 0.0260] |
| Completeness to theta 0 34.02° | (0 = 34.19°) 99.3 % | (0 = 27.93°) 97.7 % |
| Absorption correction | Multiscan | None |
| Refinement method | Full-matrix least-squares on F² | Full-matrix least-squares on F² |
| Data / restraints / parameters | 6684/0/177 | 5149/0/258 |
| Goodness-of-fit on F² | 1.028 | 2.074 |
| Final R indices [I>2sigma(I)] | R1 = 0.0398, wR2 = 0.1099 | R1 = 0.0652, wR2 = 0.2166 |
| R indices (all data) | R1 = 0.0536, wR2 = 0.1211 | R1 = 0.0743, wR2 = 0.2266 |
| Largest diff. Peak and hole | 0.480 and -0.371 e.Å⁻³ | 0.977 and -0.306 e.Å⁻³ |
| Max. and min. Transmission | 0.9593 and 0.9181 | 0.9862 and 0.8075 |

## Claims

1. A pyrene- and rylene-type compound having one of the general formulas (I) to (IV) 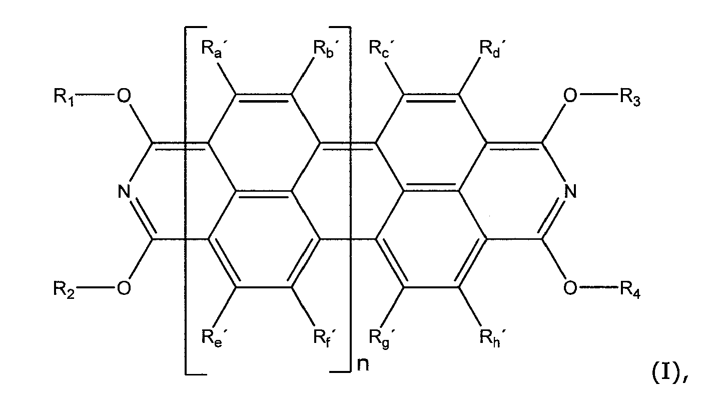 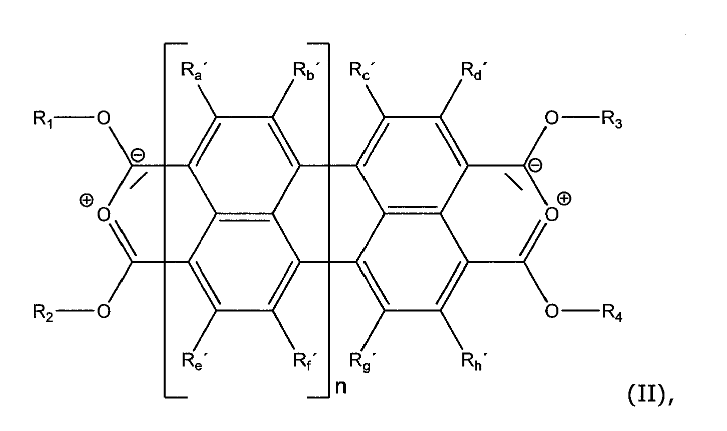 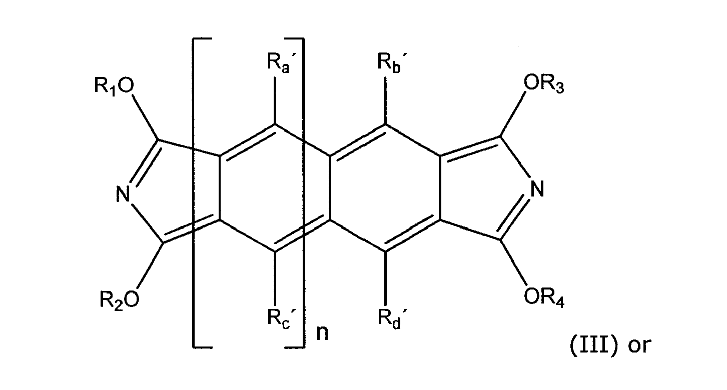 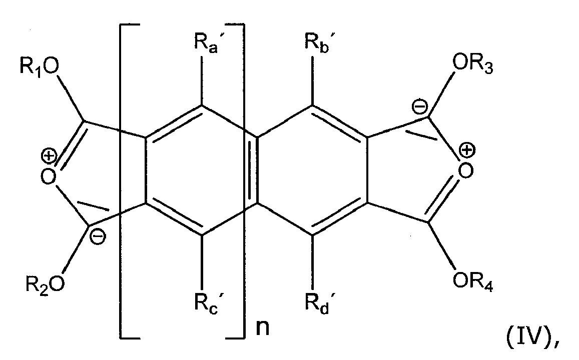 wherein
n is an integer selected from 0 to 10,
R₁ - R₄ is independently selected from an organosilyl group, an organoboryl group, an alkyl group, an aryl group, a metal ion, an organotin group, an organoalane group, an organozinc group, an organogermane group, an organogallane group, an organophosphorous group and a transition metal complex,
R_{a'} - R_{h'} is at each occurrence independently selected from H, halogen, nitro, hydroxyl, an alkyl group, an aryl group, an alkyloxy group an aryloxy group, an alkylcarbonyl group, an arylcarbonyl group, a mercaptoalkyl group, a mercaptoaryl group, a carboxy group, a carbamide group, an alkyloxycarbonyl group, a thioester group, a vinylidene group, and an arylidene group, and
Z and W shaped isomers thereof.

2. The compounds of claim 1, wherein n is 0 to 5 and R₁ - R₄ are independently selected from organosilyl groups, organoboryl groups, metal ions alkyl groups and aryl groups.

3. The compounds of claim 2, wherein
(i) n is 0, 1 or 2; and/or
(ii) R₁ - R₄ are organosilyl groups including tri-C₁₋₆alkylsilyl groups such as trimethylsilyl, triethylsilyl, triisopropyl and tri-t-butylsilyl groups, organoboryl groups including dialkyboryl and diarylboryl groups, metal ions including alkali metal ions such as lithium, sodium and potassium ions, and earth alkaline metal ionss such as magnesium and calcium ions, alkyl groups including unsubstituted and substituted, saturated and unsaturated C₁₋₁₈ alkyl groups, or aryl groups including unsubstituted and substituted C₆₋₁₄ aryls and heteroaryls; and/or
(iii) R_{a'} - R_{h'} are H, alkyl groups including unsubstituted and substituted, saturated and unsaturated C₁₋₁₈ alkyl groups, or aryl groups including unsubstituted and substituted C₆₋₁₄ aryls and heteroaryls.

4. The compound of claim 3, having the structure (I) 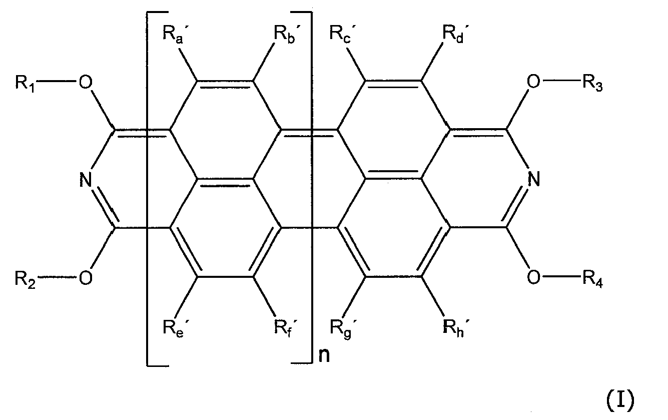 wherein n and R₁ - R₄ have the meaning as defined in claim 3 and R_{a'} - R_{h'} are H.

5. The compound of claim 4, wherein n is 0 or 1 and R₁ - R₄ are trimethylsilyl groups.

6. A method for preparing a pyrene- and rylene-type compound of formulas I to IV as defined in claims 1 to 5, which comprises
(a) reacting starting compounds of the formulas (I) to (IV), wherein R₁ - R₄ is H, or a tautomer thereof, with a strong, alkali metal-containing base under reducing conditions, and,
(b) if R₁ - R₄ are other than a metal ion, reacting the product of step (a) with a minimum of appropriate equivalents of a silylating, borylating, alkylating, arylating, organotinylating, organoalanylating, organozincylatating, organogermanylating, organogallanylating, organophosphorylating and transition metal complex-forming agent.

7. The method of claim 6, wherein
(i) the strong base is selected from alkali metals such as lithium, sodium and potassium, alkali metal amides, alcoholates, metal hydrides and complex metal hydrides, alkyl lithium and carbanionic bases,
(ii) the reducing conditions are established by use of an alkali metal;
(iii) the silylating agent is a trimethylsilylhalogenide, triethylsilylhalogenide, triisopropylsilylhalogenide, or tri-t-butylsilylhalogenide, and the borylating agent is a dialkylborylhalide or a diarylborylhalide; and/or
(iv) the reactions (a) and (b) are performed in an inert aprotic solvent, such as ethers, or, in case the base is an alkali metal amide, an amine solvent corresponding to the base;
(v) the reactions are performed at a temperature range of -78°C to ambient temperature, and even with heating under reflux of the solvent or heating in an autoclave.

8. Use of the pyrene- and rylene-type compounds of formulas (I) to (IV) as defined in claims 1 to 5 as a fluorescent dye, as detectable marker in bioassays, as constituent of supramolecular systems and of self organization systems, as reagent for electron transfer reactions and light harvesting systems, and as diagnostic tool for any species leading to a chemical degradation of the dye for a quantitative determination of such an agent.

9. Use of the pyrene- and rylene-type compounds of formulas (I) to (IV) as defined in claims 1 to 5 as intermediates for modification at positions that are vicinal to the carbon-oxygen groups of the pyrene- and rylene-type compounds of formulas (I) to (IV) or for modifying the N-atom of the compounds (I) to (II).
